# EUROPEAN PATENT SPECIFICATION

(11) **EP 1 513 589 B1**
(45) Date of publication and mention of the grant of the patent: **10.11.2010**
(21) Application number: 03727099.8
(22) Date of filing: 02.06.2003
(51) Int. Cl.: A61P 27/06, A61K 31/4015, A61K 31/41, C07D 403/12

(54) **1,5-DISUBSTITUTED PYRROLID-2-ONE DERIVATIVES FOR USE AS EP4 RECEPTOR AGONISTS IN THE TREATMENT OF EYE DISEASES SUCH AS GLAUCOMA**
1,5-DISUBSTITUIERTE PYRROLID-2-ON-DERIVATE ZUR VERWENDUNG ALS EP4 REZEPTOR AGONISTEN ZUR BEHANDLUNG VON AUGENKRANKHEITEN WIE Z.B. GLAUKOM
DERIVES DE PYRROLID-2-ONE 1,5 DISUBSTITUES UTILISES COMME AGONISTES DE RECEPTEUR EP4 POUR TRAITER DES MALADIES OCULAIRES TELLES QUE LE GLAUCOME

(30) Priority: 06.06.2002 US 386499 P; 03.04.2003 US 460134 P
(43) Date of publication of application: 16.03.2005
(73) Proprietor: Merck Frosst Canada Ltd., Kirkland, Québec H9H 3L1 (CA)
(72) Inventor: BILLOT, Xavier, Kirkland, Québec H9H 3L1 (CA); YOUNG, Robert, N., Kirkland, Québec H9H 3L1 (CA); HAN, Yongxin, Kirkland, Québec H9H 3L1 (CA)
(74) Representative: Buchan, Gavin MacNicol
(86) International application number: PCT/CA2003/000838
(87) International publication number: WO 2003/103772

(56) References cited:
- WO-A-00/38663
- WO-A-00/38667
- WO-A-02/42268
- DATABASE WPI Section Ch, Week 200247 Derwent Publications Ltd., London, GB; Class B03, AN 2002-443972 XP002252111 -& WO 02 24647 A (ONO PHARM CO LTD), 28 March 2002 (2002-03-28) cited in the application

## Description

This case claims the benefit of provisional application USSN 60/386,499, filed June 6, 2002 and USSN 60/460,134, filed April 3, 2003.

### BACKGROUND OF THE INVENTION

Glaucoma is a degenerative disease of the eye wherein the intraocular pressure is too high to permit normal eye function. As a result, damage may occur to the optic nerve head and result in irreversible loss of visual function. If untreated, glaucoma may eventually lead to blindness. Ocular hypertension, i.e., the condition of elevated intraocular pressure without optic nerve head damage or characteristic glaucomatous visual field defects, is now believed by the majority of ophthalmologists to represent merely the earliest phase in the onset of glaucoma.

Many of the drugs formerly used to treat glaucoma proved unsatisfactory. Early methods of treating glaucoma employed pilocarpine and produced undesirable local effects that made this drug, though valuable, unsatisfactory as a first line drug. More recently, clinicians have noted that many β-adrenergic antagonists are effective in reducing intraocular pressure. While many of these agents are effective for this purpose, there exist some patients with whom this treatment is not effective or not sufficiently effective. Many of these agents also have other characteristics, e.g., membrane stabilizing activity, that become more apparent with increased doses and render them unacceptable for chronic ocular use and can also cause cardiovascular effects.

Agents referred to as carbonic anhydrase inhibitors decrease the formation of aqueous humor by inhibiting the enzyme carbonic anhydrase. While such carbonic anhydrase inhibitors are now used to treat elevated intraocular pressure by systemic and topical routes, current therapies using these agents, particularly those using systemic routes are still not without undesirable effects. Topically effective carbonic anhydrase inhibitors are disclosed in U.S. Patent Nos. 4,386,098; 4,416,890; 4,426,388; 4,668,697; 4,863,922; 4,797,413; 5,378,703, 5,240,923 and 5,153,192.

Prostaglandins and prostaglandin derivatives are also known to lower intraocular pressure. There are several prostaglandin types, including the A, B, C, D, E, F, G, I and J- Series (EP 0561073 A1). U.S. Patent 4,883,819 to Bito describes the use and synthesis of PGAs, PGBs and PGCs in reducing intraocular pressure. U.S. Patent 4,824,857 to Goh et al. describes the use and synthesis of PGD₂ and derivatives thereof in lowering intraocular pressure including derivatives wherein C-10 is replaced with nitrogen. U.S. Patent 5,001,153 to Ueno et al. describes the use and synthesis of 13,14-dihydro-15-keto prostaglandins and prostaglandin derivatives to lower intraocular pressure. U.S. Patent 4,599,353 describes the use of eicosanoids and eicosanoid derivatives including prostaglandins and prostaglandin inhibitors in lowering intraocular pressure. See also WO 00/38667, WO 99/32441, WO 99/02165, WO 00/38663, WO 01/46140, EP 0855389, JP 2000-1472, US Patent No. 6,043,275 and WO 00/38690.

Prostaglandin and prostaglandin derivatives are known to lower intraocular pressure by increasing uveoscleral outflow. This is true for both the F type and A type of prostaglandins. This invention is particularly interested in those compounds that lower IOP via the uveoscleral outflow pathway and other mechanisms by which the E series prostaglandins (PGE₂) may facilitate IOP reduction. The four recognized subtypes of the EP receptor are believed to modulate the effect of lowering IOP (EP₁, EP₂, EP₃ and EP₄; J. Lipid Mediators Cell Signaling, Vol. 14, pages 83-87 (1996)). See also J. Ocular Pharmacology, Vol. 4, 1, pages 13-18 (1988); J. Ocular Pharmacology and Therapeutics, Vol. 11, 3, pages 447-454 (1995); J. Lipid Mediators, Vol. 6, pages 545-553 (1993); US Patent Nos. 5,698,598 and 5,462,968 and Investigative Ophthalmology and Visual Science, Vol. 31, 12, pages 2560-2567 (1990). Of particular interest to this invention are compounds, which are agonist of the EP₄ subtype receptor.

A problem with using prostaglandins or derivatives thereof to lower intraocular pressure is that these compounds often induce an initial increase in intraocular pressure, can change the color of eye pigmentation and cause proliferation of some tissues surrounding the eye.

As can be seen, there are several current therapies for treating glaucoma and elevated intraocular pressure, but the efficacy and the side effect profiles of these agents are not ideal. Therefore, there still exist the need for new and effective therapies with little or no side effects.

A variety of disorders in humans and other mammals involve or are associated with abnormal or excessive bone loss. Such disorders include, but are not limited to, osteoporosis, glucocorticoid induced osteoporosis, Paget's disease, abnormally increased bone turnover, periodontal disease, tooth loss, bone fractures, rheumatoid arthritis, periprosthetic osteolysis, osteogenesis imperfecta, metastatic bone disease, hypercalcemia of malignancy, and multiple myeloma. One of the most common of these disorders is osteoporosis, which in its most frequent manifestation occurs in postmenopausal women. Osteoporosis is a systemic skeletal disease characterized by a low bone mass and microarchitectural deterioration of bone tissue, with a consequent increase in bone fragility and susceptibility to fracture. Osteoporotic fractures are a major cause of morbidity and mortality in the elderly population. As many as 50% of women and a third of men will experience an osteoporotic fracture. A large segment of the older population already has low bone density and a high risk of fractures. There is a significant need to both prevent and treat osteoporosis and other conditions associated with bone resorption. Because osteoporosis, as well as other disorders associated with bone loss, are generally chronic conditions, it is believed that appropriate therapy will typically require chronic treatment.

Two different types of cells called osteoblasts and osteoclasts are involved in the bone formation and resorption processes, respectively. *See* H. Fleisch, Bisphosphonates In Bone Disease, From The Laboratory To The Patient, 3rd Edition, Parthenon Publishing (1997), which is incorporated by reference herein in its entirety. Osteoblasts are cells that are located on the bone surface. These cells secrete an osseous organic matrix, which then calcifies. Substances such as fluoride, parathyroid hormone, and certain cytokines such as protaglandins are known to provide a stimulatory effect on osetoblast cells. However, an aim of current research is to develop therapeutic agents that will selectively increase or stimulate the bone formation activity of the osteoblasts.

Osteoclasts are usually large multinucleated cells that are situated either on the surface of the cortical or trabecular bone or within the cortical bone. The osteoclasts resorb bone in a closed, sealed-off microenvironment located between the cell and the bone. The recruitment and activity of osteoclasts is known to be influenced by a series of cytokines and hormones. It is well known that bisphosphonates are selective inhibitors of osteoclastic bone resorption, making these compounds important therapeutic agents in the treatment or prevention of a variety of systemic or localized bone disorders caused by or associated with abnormal bone resorption. However, despite the utility of bisphosphonates there remains the desire amongst researchers to develop additional therapeutic agents for inhibiting the bone resorption activity of osteoclasts.

Prostaglandins such as the PGE₂ series are known to stimulate bone formation and increase bone mass in mammals, including man. It is believed that the four different receptor subtypes, designated EP₁, EP₂, EP₃, and EP₄ are involved in mediating the bone modeling and remodeling processes of the osteoblasts and osteoclasts. The major prostaglandin receptor in bone is EP₄, which is believed to provide its effect by signaling via cyclic AMP.

In present invention it is further found that the formula I agonists of the EP₄ subtype receptor are useful for stimulating bone formation.

WO 02/24647, WO 02/42268, EP 1132086, EP 855389, EP 1114816, WO 01/46140 and WO 01/72268 disclose EP₄ agonists. However, they do not disclose the compounds of the instant invention.

### SUMMARY OF THE INVENTION

This invention relates to potent selective agonists of the EP₄ subtype of prostaglandin E2 receptors, their use or a formulation thereof in the treatment of glaucoma and other conditions that are related to elevated intraocular pressure in the eye of a patient.

More particularly, this invention relates to the use, for the manufacture of a medicament for treating ocular hypertension or glaucoma, of a compound of formula I: 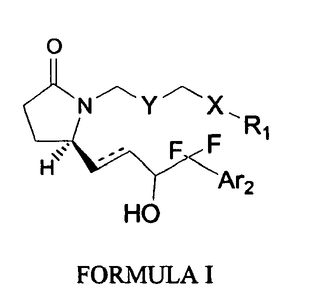 or a pharmaceutically acceptable salt, enantiomer, diastereomer, or mixture thereof, wherein,
X is (CH₂)ₙ, O or S;
Y represents (C(R^{b})₂)ₙ, triple bond, 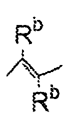 or 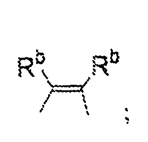 R₁ represents tetrazole which is unsubstituted or substituted with an Ra group, or (CH₂)ₚCO₂R₆, with the proviso that when X is a bond R₁ is not (CH₂)ₚCO₂R₆.
R² and R³ independently represent hydrogen, or C₁₋₄ alkyl;
R₆ represents hydrogen, or C₁₋₆ alkyl;
Ar₂ independently represents (CH₂)ₘC₆₋₁₀aryl, (CH₂)ₘC₅₋₁₀heteroaryl, (CH₂)ₘC₃₋₁₀ heterocycloalkyl, (CH₂)ₘC₃₋₈ cycloalkyl said cycloalkyl, heterocycloalkyl, aryl or heteroaryl unsubstituted or substituted with 1-3 groups of Rₐ;
Rₐ represents C₁₋₆ alkoxy, C₁₋₆ alkyl, CF₃, nitro, amino, cyano, C₁₋₆ alkylamino, or halogen;
R^{b} independently represents H, halogen, C₁₋₆ alkyl, or C₃₋₆ cylcoalkyl;
--- represents a double or single bond;
p represents 1-3;
n represents 0-4; and
m represent 0-8.

This and other aspects of the invention will be realized upon inspection of the invention as a whole.

### DETAILED DESCRIPTION OF THE INVENTION

The invention is described herein in detail using the terms defined below unless otherwise specified.

The term "therapeutically effective amount", as used herein, means that amount of the EP₄ receptor subtype agonist of formula I, or other actives of the present invention, that will elicit the desired therapeutic effect or response or provide the desired benefit when administered in accordance with the desired treatment regimen. A preferred therapeutically effective amount relating to the treatment of abnormal bone resorption is a bone formation, stimulating amount. Likewise, a preferred therapeutically effective amount relating to the treatment of ocular hypertension or glaucoma is an amount effective for reducing intraocular pressure and/or treating ocular hypertension and/or glaucoma.

"Pharmaceutically acceptable" as used herein, means generally suitable for administration to a mammal, including humans, from a toxicity or safety standpoint.

The term "alkyl" refers to a monovalent alkane (hydrocarbon) derived radical containing from 1 to 10 carbon atoms unless otherwise defined. It may be straight or branched. Preferred alkyl groups include methyl, ethyl, propyl, isopropyl, butyl and t-butyl. When the alkyl group is said to be substituted with an alkyl group, this is used interchangeably with "branched alkyl group".

Alkoxy refers to C₁-C₆ alkyl-O-, with the alkyl group optionally substituted as described herein. Examples of alkoxy groups are methoxy, ethoxy, propoxy, butoxy and isomeric groups thereof.

Halogen (halo) refers to chlorine, fluoride, iodine or bromine. Aryl refers to aromatic rings e.g., phenyl, substituted phenyl and the like, as well as rings which are fused, e.g., naphthyl, phenanthrenyl and the like. An aryl group thus contains at least one ring having at least 6 atoms, with up to five such rings being present, containing up to 22 atoms therein, with alternating (resonating) double bonds between adjacent carbon atoms or suitable heteroatoms. The preferred aryl groups are phenyl, naphthyl and phenanthrenyl, Aryl groups may likewise be substituted as defined. Preferred substituted aryls include phenyl and naphthyl.

The term "heterocycloalkyl" refers to a cycloalkyl group (nonaromatic) having 3 to 10 carbon atoms in which one of the carbon atoms in the ring is replaced by a heteroatom selected from O, S or N, and in which up to three additional carbon atoms may be replaced by hetero atoms.

The term "cycloalkyl" refers to a cyclic alkyl group (nonaromatic) having 3 to 10 carbon atoms.

The term "heteroatom" means O, S or N, selected on an independent basis.

The term "heteroaryl" refers to a monocyclic aromatic group having 5 or 6 ring atoms, or a bicyclic aromatic group having 8 to 10 atoms, containing at least one heteroatom, O, S or N, in which a carbon or nitrogen atom is the point of attachment, and in which one or two additional carbon atoms is optionally replaced by a heteroatom selected from O or S, and in which from 1 to 3 additional carbon atoms are optionally replaced by nitrogen heteroatoms, said heteroaryl group being optionally substituted as described herein. Examples of this type are pyrrole, pyridine, oxazole, thiazole, tetrazole, and oxazine. For purposes of this invention the tetrazole includes all tautomeric forms. Additional nitrogen atoms may be present together with the first nitrogen and oxygen or sulfur, giving, e.g., thiadiazole.

The term heterocyclyl or heterocyclic, as used herein, represents a stable 5- to 7-membered monocyclic or stable 8- to 11-membered bicyclic heterocyclic ring which is either saturated or unsaturated, and which consists of carbon atoms and from one to four heteroatoms selected from the group consisting of N, O, and S, and including any bicyclic group in which any of the above-defined heterocyclic rings is fused to a benzene ring. The heterocyclic ring may be attached at any heteroatom or carbon atom which results in the creation of a stable structure. A fused heterocyclic ring system may include carbocyclic rings and need include only one heterocyclic ring. The term heterocycle or heterocyclic includes heteroaryl moieties. Examples of such heterocyclic elements include, but are not limited to, azepinyl, benzimidazolyl, benzisoxazolyl, benzofurazanyl, benzopyranyl, benzothiopyranyl, benzofuryl, benzothiazolyl, benzothienyl, benzoxazolyl, chromanyl, cinnolinyl, dihydrobenzofuryl, dihydrobenzothienyl, dihydrobenzothiophenyl, dihydrobenzothiopyranyl sulfone, 1,3-dioxolanyl, furyl, imidazolidinyl, imidazolinyl, imidazolyl, indolinyl, indolyl, isochromanyl, isoindolinyl, isoquinolinyl, isothiazolidinyl, isothiazolyl, isothiazolidinyl, morpholinyl, naphthyridinyl, oxadiazolyl, 2-oxoazepinyl, oxazolyl, 2-oxopiperazinyl, 2-oxopiperdinyl, 2-oxopyrrolidinyl, piperidyl, piperazinyl, pyridyl, pyrazinyl, pyrazolidinyl, pyrazolyl, pyridazinyl, pyrimidinyl, pyrrolidinyl, pyrrolyl, quinazolinyl, quinolinyl, quinoxalinyl, tetrahydrofuryl, tetrahydroisoquinolinyl, tetrahydroquinolinyl, thiamorpholinyl, thiamorpholinyl sulfoxide, thiazolyl, thiazolinyl, thienofuryl, thienothienyl, and thienyl. An embodiment of the examples of such heterocyclic elements include, but are not limited to, azepinyl, benzimidazolyl, benzisoxazolyl, benzofurazanyl, benzopyranyl, benzothiopyranyl, benzofuryl, benzothiazolyl, benzothienyl, benzoxazlyl, chromanyl, cinnolinyl, dihydrobenzofuryl, dihydrobenzothienyl, dihydrobenzothiopyranyl, dihydrobenzothiopyranyl sulfone, furyl, imidazolidinyl, imidazolinyl, imidazolyl, indolinyl, indolyl, isochromanyl, isoindolinyl, isoquinolinyl, isothiazolidinyl, isothiazolyl, isothiazolidinyl, morpholinyl, naphthyridinyl, oxadiazolyl, 2-oxoazepinyl, oxazolyl, 2-oxopiperazinyl, 2-oxopiperdinyl, 2-oxopyrrolidinyl, piperidyl, piperazinyl, pyridyl, 2-pyridinonyl, pyrazinyl, pyrazolidinyl, pyrazolyl, pyridazinyl, pyrimidinyl, pyrrolidinyl, pyrrolyl, quinazolinyl, quinolinyl, quinoxalinyl, tetrahydrofuryl, tetrahydroisoquinolinyl, tetrahydroquinolinyl, thiamorpholinyl, thiamorpholinylsulfoxide, thiazolyl, thiazolinyl, thienofuryl, thienothienyl, thienyl and triazolyl.

The term "agonist" as used herein means EP₄ subtype compounds of formula I interact with the EP4 receptor to produce maximal, super maximal or submaximal effects compared to the natural agonist, PGE2. See Goodman and Gilman. The Pharmacological Basis of Therapeutics, 9th edition, 1996, chapter 2.

One embodiment of this invention is realized when X is (CH₂)ₙ,. Another embodiment of this invention is when X is Sulfur. Still another embodiment of this invention is when X is oxygen.

Another embodiment of this invention is realized when Y is (CH₂)ₙ and all other variables are as originally described.

Still another embodiment of this invention is realized when Y is C(halo)₂ and all other variables are as originally described.

Still another embodiment of this invention is realized when Y is a double bond as described by 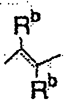 or 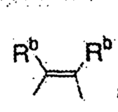 and all other variables are as originally described.

Still another embodiment of this invention is realized when Y is a triple bond and all other variables are as originally described.

Another embodiment of this invention is realized when R₁ is (CH₂)ₚCO₂R₆, and all other variables are as originally described. A subembodiment of this invention is realized when X is (CH₂)ₙ, and Y is (CH₂)ₙ.

Another embodiment of this invention is realized when Ar₂ is (CH₂)ₘC₆₋₁₀aryl, said aryl unsubstituted or substituted with 1 to 3 groups of R^{a} and all other variables are as originally described,

Still another embodiment of this invention is realized when R₁ is a tetrazole unsubstituted or substituted with an Rₐ group and all other variables are as originally described. A subembodiment of this invention is realized when X is (CH₂)ₙ, and Y is (CH₂)ₙ, C(halo)₂ or a double bond as described by 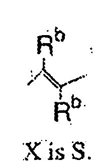 or 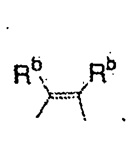 Another subembodiment of this invention is realized when

Still another embodiment of this invention is realized, when Ar₂ is a phenyl unsubstituted or substituted with 1 to 3 groups of Rₐ and all other variables are as originally described.

Yet another embodiment of this invention is realized when R₁ is tetrazolyl and Ar₂ is phenyl, said tetrazolyl unsubstituted or substituted with a Rₐ group and phenyl is unsubstituted or substituted with 1-3 groups of Rₐ, and all other variables are as originally described. A subembodiment of this invention is realized when X is (CH₂)ₙ, and Y is (CH₂)ₙ, C(halo)₂ or a double bond as described by 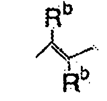 or 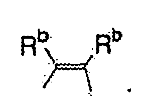 Another subembodiment of this invention is realized when X is sulfur.

Still another embodiment of this invention is realized when a compound of formula II: 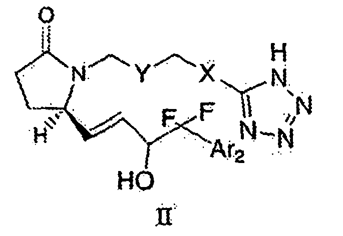 or a pharmaceutically acceptable salt, enantiomer, diastereomer, or mixture thereof is used, wherein X, Y, Ar₂ and n are as previously described.

A subembodiment of this invention is realized when X is (CH₂)ₙ, and Y is (CH₂)ₙ, C(halo)₂ or a double bond as described by 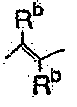 or 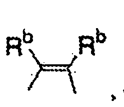 and Ar₂ is phenyl. Another subembodiment of this invention is realized when n is 4.

A subembodiment of this invention is realized when X is S.

Compound used in this invention are:
(5*R*)-5-[(1*E*)-3-hydroxy-4,4-difluoro-4-phenylbut-1-enyl]-1-[4-[(1-methyl-1*H-*tetrazol-5-yl)]butyl}pyrrolidin-2-one,
3-[4-{(2*R*)-2-[(1*E*)-3-hydroxy-4,4-difluoro-4-phenylbut-1-enyl]-5-pyrrolidin-1-yl}butyl)]propanoic acid,
(5*R*)-5-(1*E*)-3-hydroxy-4,4-difluoro-4-phenylbut-1-enyl]-1-{4-[1*H*-tetrazol-5-ylmethyl)butyl}pyrrolidin-2-one,
[4-{(2*R*)-2-[(1*E*)-3-hydroxy-4,4-difluoro-4-phenylbut-1-enyl]-5-oxopyrrolidin-2-yl}bulyl)]acetic acid,
(5*R*)-5-[(1*E*)-3-hydroxy-4,4-difluoro-4-phenylbut-1-enyl]-1-{4-[(1-methyl-1*H-*tetrazol-5-yl)thio]butyl}pyrrolidin-2-one,
(5*R*)-5-[(1*E*)-3-hydroxy-4,4-difluoro-4-phenylbut-1-enyl]-1-[4-(1*H*-tetrazol-5-ylthio)butyl]pyrrolidin-2-one;
3-[4-{(2*R*)-2-[(1*E*)-3-hydroxy-4,4-difluoro-4-phenylbut-1-enyl]-5-oxopyrrolidin-1-yl}butyl)thio]propanoic acid,
[4-{(2*R*)-2-[(1*E*)-3-hydroxy-4,4-difluoro-4-phenylbut-1-enyl]-5-oxopyrrolidon-1-yl}butyl)thio]acetic acid,
(5R)-5[(1E)-4,4-difluoro-3-hydroxy-4-phenylbut-1-enyl]-1-[6-(1H-tetrazol-5-yl)hexyl]pyrrolidin-2-one,
7-{(2*R*)-2-[(1*E*,3*R*)-4,4-difluoro-3-hydroxy-4-phenylbut-1-enyl]-5-oxopyrrolidin-1-yl}heptanoic acid,
isopropyl 7-{(2*R*)-2-[(1*E*,3*R*)-4,4-difluoro-3-hydroxy-4-phenylbut-1-enyl]-5-oxopyrrolidin-1-yl}heptanoate,
7-{(2*S*)-2-[(3*R*)-4,4-difluoro-3-hydroxy-4-phenylbutyl]-5-oxopyrrolidin-1-yl}heptanoic acid,
(5Z)-7-{(2*R*)-2-[(1*E*,3*R*)-4,4-difluoro-3-hydroxy-4-phenylbut-1-enyl]-5-oxopyrrolidin-1-yl}hept-5-enoic acid,
isopropyl (5*Z*)-7-{(2*R*)-2-[(1*E*,3*R*)-4,4-difluoro-3-hydroxy-4-phenylbut-1-enyl]-5-oxopyrrolidin-1-yl}hept-5-enoate,
7-{(2*R*)-2-[(1*E*,3*R*)-4-(3-chlorophenyl)-4,4-difluoro-3-hydroxybut-1-enyl]-5-oxopyrrolidin-1-yl}heptanoic acid,
isopropyl 7-{(2*R*)-2-[(1*E*,3*R*)-4-(3-chlorophenyl)-4,4-difluoro-3-hydroxybut-1-enyl]-5-oxopyrrolidin-1-yl}heptanoate;
7-((2*R*)-2-{(1*E*,3*R*)-4,4-difluoro-3-hydroxy-4-[3-(trifluoromethyl)phenyl]but-1-enyl}-5-oxopyrrolidin-1-yl)heptanoic acid,
isopropyl 7-((2*R*)-2-{(1*E*,3*R*)-4,4-difluoro-3-hydroxy-4-[3-(trifluoromethyl)phenyl]but-1enyl}-5-oxopyrrolidin-1-yl)heptanoate,
cyclopentyl 7-{(2*R*)-2-[(1*E*,3*R*)-4,4-difluoro-3-hydroxy-4-phenylbut-1-enyl]-5-oxopyrrolidin-1-yl}heptanoate,
7-{(2*R*)-2-[(1*E*,3*R*)-4,4-difluoro-3-hydroxy-3-methyl-4-phenylbut-1-enyl]-5-oxopyrrolidin-1-yl}heptanoic acid,
isopropyl 7-{(2*R*)-2-[(1*E*,3*R*)-4,4-difluoro-3-hydroxy-3-methyl-4-phenylbut-1-enyl]-5-oxopyrrolidin-1-yl}heptanoate,
isobutyl 7-{(*R*)-2-[(1*E*,3*R*)-4,4-difluoro-3-hydroxy-4-phenylbut-1-enyl]-5-oxopyrrolidin-1-yl}heptanoate, and
cyclohexyl 7-{(2*R*)-2-[(1*E*,3*R*)-4,4-difluoro-3-hydroxy-4-phenylbut-1-enyl]-5-oxopyrrolidin-1-yl}heptanoate, or a pharmaceutically acceptable salt, enantiomer, diastereomer, or mixture thereof.

Additional compounds are:
(5*R*)-5-[(1*E*)-(3R)-hydroxy-4,4-difluoro-4-phenylbut-1-enyl]-1-{4-[(1-methyl-1*H-*tetrazol-5-yl)]butyl}pyrrolidin-2-one;
3-[4-{(2*R*)-2-(1*E*)-(3R)-hydroxy-4,4-difluoro-4-phenylbut-1-enyl]-5oxopyrrolidin-1-yl}butyl)]propanoic acid,
(5*R*)-5-[(1*E*)-(3R)-hydroxy-4,4-difluoro-4-phenylbut-1-enyl]-1-{4-[1*H*-tetrazol-5-ylmethyl)butyl}pyrrolidin-2-one,
[4-{(2*R*)-2-[(1*E*)-(3R)-hydroxy-4,4-difluoro-4-phenylbut-1-enyl]-5-oxopyrrolodin-1-yl}butyl)]acetic acid,
(5*R*)-5-[(1*E*)-(3R)-hydroxy-4,4-difluoro-4-phenylbut-1-enyl]-1-{4-[(1-methyl-1*H-*tetrazol-5-yl)thio]butyl}pyrrolidin-2-one,
(5*R*)-5-[(1*E*)-(3R)-hydroxy-4,4-difluoro-4-phenylbut-1-enyl]-1-[4-(1*H*-tetrazol-5-ylthio)butyl]pyrrolidin-2-one,
3-[4-{(2*R*)-2-[(1*E*)-(3R)-hydroxy-4,4-difluoro-4-phenylbut-1-enyl]-5-oxopyrrolidin-1-yl]butyl)thio]propanoic acid,
[4-{(2*R*)-2-[(1*E*)-(3R)-hydroxy-1,4-difluoro-4-phenylbut-1-enyl]-5-oxopyrrolidin-1-yl}butyl)thio]acetic acid, or
(5R)-5[(1E)-4,4-difluoro-(3R)-hydroxy-4-phenylbut-1-enyl]-1-[6-(1H-tetrazol-5-yl)hexyl]pyrrolidin-2-one, a pharmaceutically acceptable salt, enantiomer, diastereomer, or mixture thereof.

Another embodiment of this invention is directed to a composition containing an EP₄ agonist of Formula I and optionally a pharmaceutically acceptable carrier.

This invention is further concerned with a process for making a pharmaceutical composition comprising a compound of formula I.

This invention is further concerned with a process for making a pharmaceutical composition comprising a compound of formula I, and a pharmaceutically acceptable carrier.

The claimed compounds bind strongly and act on PGE₂ receptor, particularly on the EP₄ subtype receptor and therefore are useful for preventing and/or treating glaucoma and ocular hypertension.

Dry eye is a common ocular surface disease afflicting millions of people. Although it appears that dry eye may result from a number of unrelated pathogenic causes, the common end result is the breakdown of the tear film, which results in dehydration of the exposed outer surface of the eye. (Lemp, Report of the Nation Eye Institute/Industry Workshop on Clinical Trials in Dry Eyes, The CLAO Journal, 21(4):221-231 (1995)). One cause for dry eye is the decreased mucin production by the conjunctival cells and/or corneal epithelial cells of mucin, which protects and lubricates the ocular surface (Gipson and Inatomi, Mucin genes expressed by ocular surface epithelium. Progress in Retinal and Eye Research, 16:81-98 (1997)). Functional EP4 receptors have been found in human conjuctival epithelial cells (see US Patent 6,344,477, incorporated by reference in its entirey) and it is appreciated that both human corneal epithelial cells (Progess in Retinal and Eye Research, 16:81-98(1997)) and conjuctival cells (Dartt et al. Localization of nerves adjacent to goblet cells in rat conjucntiva. Current Eye Research, 14:993-1000 (1995)) are capable of secreting mucins. Thus, the compounds of formula I are useful for treating dry eye.

Macular edema is swelling within the retina within the critically important central visual zone at the posterior pole of the eye. An accumulation of fluid within the retina tends to detach the neural elements from one another and from their local blood supply, creating a dormancy of visual function in the area. It is believed that EP₄ agonist which lower IOP are useful for treating diseases of the macular such as macular edema or macular degeneration. Thus, another aspect of this invention is a method for treating macular edema or macular degeneration.

Glaucoma is characterized by progressive atrophy of the optic nerve and is frequently associated with elevated intraocular pressure (IOP). It is possible to treat glaucoma, however, without necessarily affecting IOP by using drugs that impart a neuroprotective effect. See Arch. Ophthalmol. Vol. 112, Jan 1994, pp. 37-44; Investigative Ophthamol. & Visual Science, 32, 5, April 1991, pp. 1593-99. It is believed that EP₄ agonist which lower IOP are useful for providing a neuroprotective effect. They are also believed to be effective for increasing retinal and optic nerve head blood velocity and increasing retinal and optic nerve oxygen by lowering IOP, which when coupled together benefits optic nerve health. As a result, this invention further relates to a method for increasing retinal and optic nerve head blood velocity, or increasing retinal and optic nerve oxygen tension or providing a neuroprotective effect or a combination thereof by using an EP₄ agonist of formula I.

The compounds produced in the present invention are readily combined with suitable and known pharmaceutically acceptable excipients to produce compositions which may be administered to mammals, including humans, to achieve effective IOP lowering. Thus, this invention enables a method of treating ocular hypertension or glaucoma by administering to a patient in need thereof one of the compounds of formula I alone or in combination with a β-adrenergic blocking agent such as timolol, betaxolol, levobetaxolol, carteolol, levobunolol, a parasympathomimetic agent such as pilocarpine, a sympathomimetic agents such as epinephrine, iopidine, brimonidine, clonidine, para-aminoclonidine, a carbonic anhydrase inhibitor such as dorzolamide, acetazolamide, metazolamide or brinzolamide; a Maxi-K channel blocker as disclosed in USSN 60/389,205, filed June 17, 2002, 60/389,222, filed June 17, 2002, 60/458,981, filed March 27, 2003, 60/424790, filed November 8, 2002, 60/424808, filed November 8, 2002, 09/765716, filed January 17, 2001, 09/764738, filed January 17, 2001 and PCT publications WO 02/077168 and WO 02/02060863, a prostaglandin such as latanoprost, travaprost, unoprostone, rescula, S 1033 (compounds set forth in US Patent Nos. 5,889,052; 5,296,504; 5,422,368; and 5,151,444); a hypotensive lipid such as lumigan and the compounds set forth in US Patent No. 5,352,708; a neuroprotectant disclosed in US Patent No. 4,690,931, particularly eliprodil and R-eliprodil as set forth in WO 94/13275, including memantine; or an agonist of 5-HT2 receptors as set forth in PCT/US00/31247, particularly 1-(2-aminopropyl)-3-methyl-1H-imdazol-6-ol fumarate and 2-(3-chloro-6-methoxy-indazol-1-yl)-1-methyl-ethylamine.

This invention also enables a method for increasing retinal and optic nerve head blood velocity, or increasing retinal and optic nerve oxygen tension or providing a neuroprotective effect or a combination thereof by administering to a patient in need thereof one of the compounds of formula I alone or in combination with a β-adrenergic blocking agent such as timolol, betaxolol, levobetaxolol, carteolol, levobunolol, a parasympathomimetic agent such as pilocarpine, a sympathomimetic agents such as epinephrine, iopidine, brimonidine, clonidine, para-aminoclonidine, a carbonic anhydrase inhibitor such as dorzolamide, acetazolamide, metazolamide or brinzolamide; a Maxi-K channel blocker as described above; a prostaglandin such as latanoprost, travaprost, unoprostone, rescula, S1033 (compounds set forth in US Patent Nos. 5,889,052; 5,296,504; 5,422,368; and 5,151,444); a hypotensive lipid such as lumigan and the compounds set forth in US Patent No. 5,352,708; a neuroprotectant disclosed in US Patent No. 4,690,931, particularly eliprodil and R-eliprodil as set forth in WO 94/13275, including memantine; or an agonist of 5-HT2 receptors as set forth in PCT/US00/31247, particularly 1-(2-aminopropyl)-3-methyl-1H-imdazol-6-ol fumarate and 2-(3-chloro-6-methoxy-indazol-1-yl)-1-methyl-ethylamine. Use of the compounds of formula I for the manufacture of a medicament for increasing retinal and optic nerve head blood velocity, or increasing retinal and optic nerve oxygen tension or providing a neuroprotective effect or a combination thereof is also included in this invention.

This invention further enables a method for treating macular edema or macular degeneration by administering to a patient in need thereof one of the compounds of formula I alone or in combination with a β-adrenergic blocking agent such as timolol, betaxolol, levobetaxolol, carteolol, levobunolol, a parasympathomimetic agent such as pilocarpine, a sympathomimetic agents such as epinephrine, iopidine, brimonidine, clonidine, para-aminoclonidine, a carbonic anhydrase inhibitor such as dorzolamide, acetazolamide, metazolamide or brinzolamide; a Maxi-K channel blocker as described above, a prostaglandin such as latanoprost, travaprost, unoprostone, rescula, S1033 (compounds set forth in US Patent Nos. 5,889,052; 5,296,504; 5,422,368; and 5,151,444); a hypotensive lipid such as lumigan and the compounds set forth in US Patent No. 5,352,708; a neuroprotectant disclosed in US Patent No. 4,690,931, particularly eliprodil and R-eliprodil as set forth in WO 94/13275, including memantine; or an agonist of 5-HT2 receptors as set forth in PCT/US00/31247, particularly 1-(2-aminopropyl)-3-methyl-1H-imdazol-6-ol fumarate and 2-(3-chloro-6-methoxy-indazol-1-yl)-1-methyl-ethylamine. Use of the compounds of formula I for the manufacture of a medicament for macular edema or macular degeneration is also included in this invention.

The EP₄ agonist used in the instant invention can be administered in a therapeutically effective amount intravaneously, subcutaneously, topically, transdermally, parenterally or any other method known to those skilled in the art. Ophthalmic pharmaceutical compositions are preferably adapted for topical administration to the eye in the form of solutions, suspensions, ointments, creams or as a solid insert. Ophthalmic formulations of this compound may contain from 0.001 to 5% and especially 0.001 to 0.1% of medicament. Higher dosages as, for example, up to about 10% or lower dosages can be employed provided the dose is effective in reducing intraocular pressure, treating glaucoma, increasing blood flow velocity or oxygen tension. For a single dose, from between 0.001 to 5.0 mg, preferably 0.005 to 2.0 mg, and especially 0.005 to 1.0 mg of the compound can be applied to the human eye.

The pharmaceutical preparation which contains the compound may be conveniently admixed with a non-toxic pharmaceutical organic carrier, or with a non-toxic pharmaceutical inorganic carrier. Typical of pharmaceutically acceptable carriers are, for example, water, mixtures of water and water-miscible solvents such as lower alkanols or aralkanols, vegetable oils, peanut oil, polyalkylene glycols, petroleum based jelly, ethyl cellulose, ethyl oleate, carboxymethyl-cellulose, polyvinylpyrrolidone, isopropyl myristate and other conventionally employed acceptable carriers. The pharmaceutical preparation may also contain non-toxic auxiliary substances such as emulsifying, preserving, wetting agents, bodying agents and the like, as for example, polyethylene glycols 200, 300, 400 and 600, carbowaxes 1,000, 1,500, 4,000, 6,000 and 10,000, antibacterial components such as quaternary ammonium compounds, phenylmercuric salts known to have cold sterilizing properties and which are non-injurious in use, thimerosal, methyl and propyl paraben, benzyl alcohol, phenyl ethanol, buffering ingredients such as sodium borate, sodium acetates, gluconate buffers, and other conventional ingredients such as sorbitan monolaurate, triethanolamine, oleate, polyoxyethylene sorbitan monopalmitylate, dioctyl sodium sulfosuccinate, monothioglycerol, thiosorbitol, ethylenediamine tetracetic acid, and the like. Additionally, suitable ophthalmic vehicles can be used as carrier media for the present purpose including conventional phosphate buffer vehicle systems, isotonic boric acid vehicles, isotonic sodium chloride vehicles, isotonic sodium borate vehicles and the like. The pharmaceutical preparation may also be in the form of a microparticle formulation. The pharmaceutical preparation may also be in the form of a solid insert. For example, one may use a solid water soluble polymer as the carrier for the medicament. The polymer used to form the insert may be any water soluble non-toxic polymer, for example, cellulose derivatives such as methylcellulose, sodium carboxymethyl cellulose, (hydroxyloweralkyl cellulose), hydroxyethyl cellulose, hydroxypropyl cellulose, hydroxypropylmethyl cellulose; acrylates such as polyacrylic acid salts, ethylacrylates, polyactylamides; natural products such as gelatin, alginates, pectins, tragacanth; karaya, chondrus, agar, acacia; the starch derivatives such as stanch acetate, hydroxymethyl starch ethers, hydroxypropyl starch, as well as other synthetic derivatives such as polyvinyl alcohol, polyvinyl pyrrolidone, polyvinyl methyl ether, polyethylene oxide, neutralized carbopol and xanthan gum, gellan gum, and mixtures of said polymer.

Suitable subjects for the administration of the formulation of the present invention include primates, man and other animals, particularly man and domesticated animals such as cats, rabbits and dogs,

The pharmaceutical preparation may contain non-toxic auxiliary substances such as antibacterial components which are non-injurious in use, for example, thimerosal, benzalkonium chloride, methyl and propyl paraben, benzyldodecinium bromide, benzyl alcohol, or phenylethanol; buffering ingredients such as sodium chloride, sodium borate, sodium acetate, sodium citrate, or gluconate buffers; and other conventional ingredients such as sorbitan monolaurate, triethanolamine, polyoxyethylene sorbitan monopalmitylate, ethylenediamine tetraacetic acid, and the like.

The ophthalmic solutions or suspension may be administered as often as necessary to maintain an acceptable IOP level in the eye. It is contemplated that adininistration to the mammalian eye will be from once up to three times daily.

For topical ocular administration the novel formulations of this invention may take the form of solutions, gels, ointments, suspensions or solid inserts, formulated so that a unit dosage comprises a therapeutically effective amount of the active component or some multiple thereof in the case of a combination therapy.

Regarding treatment of ocular disorders, the formula I agonists generally have an Ec₅₀ value from about 0.001 nM to about. 100 microM, although agonists with activities outside this range can be useful depending upon the dosage and route of administration. In a subclass of the present invention, the agonists have an EG₅₀ value of from about 0.01 microM to about 10 microM. In a further subclass of the present invention, the agonists have an EC₅₀ value of from about 0.1 microM to about 10 microM. EC₅₀ is a common measure of agonist activity well known to those of ordinary skill in the art and is defined as the concentration or dose of an agonist that is needed to produce half, i.e. 50%, of the maximal effect. See also, Goodman and Gilman's, The Pharmacologic Basis of Therapeutics, 9th edition, 1996, chapter 2, E. M. Ross, Pharmacodynamics, Mechanisms of Drug Action and the Relationship Between Drug Concentration and Effect*,* and PCT US99/23757, filed October 12,1999.

The compounds or this invention can be made, with some modification, in accordance with US Patent No. 6,043,275, EP0855389, USSN 60/337228 (Merck Docket No. MC052) and WO 01/46140. The following non-limiting examples, given by way of Illustration, is demonstrative of the present invention.

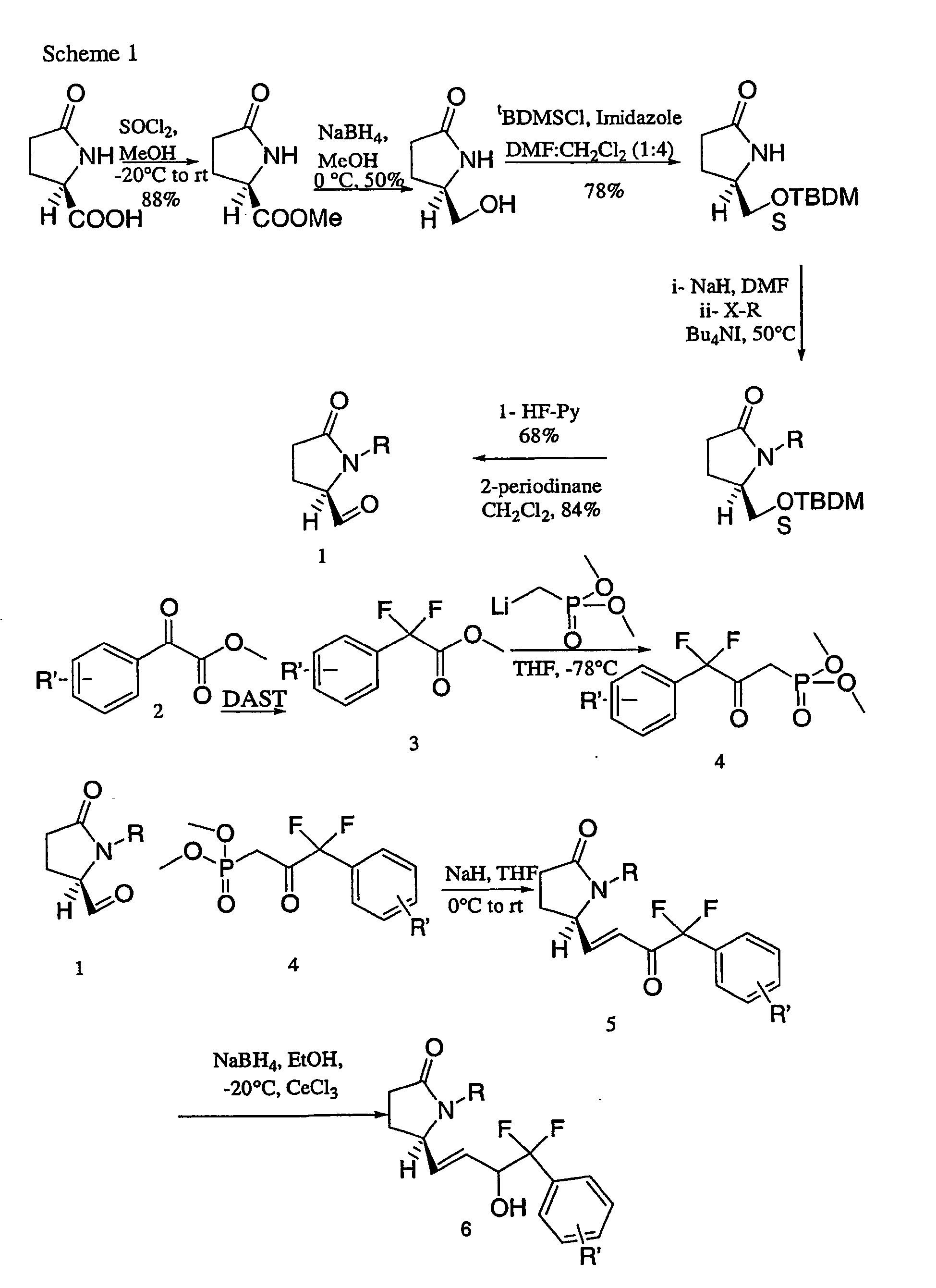

The compounds embodied in this application may be synthesized in part by Scheme 1. Pyroglutamic acid is converted to the corresponding ester through the action of a dehydrating agent such as thienylchloride and an alcohol such as methanol. The ester can be reduced by a reducing agent such as sodium borohydrate to provide the corresponding alcohol. Protection of the alcohol with a suitable protecting group such as t-butyldimethylsilylchloride in the presence of a base such as imidazole provides these silyl-protected alcohol. Reacting the protected alcohol amide with a strong base such as sodium hydride and thence with an alkyl halide (RX) provides the N-alkylated product. Alternatively, a protecting group such as paramethoxybenzylbromide may be substituted for RX to provide a protected analog of the N-alkylated product. Removal of the silyl-protecting group with a reagent such as HF-pyridine and then subsequent oxidation of the free alcohol with an oxidizing agent such as 2-periodinane then provides the aldehyde 1.

An aryl ketoester such as 2 is reacted with a fluoronating agent such as DAST to provide the difluoro ester 3. Reaction with lithiomethylene dimethoxyphosphonate provides the ketophosphonate 4. Reaction of the aldehyde 1 with the ketophosphonate 4 in the presence of a strong base such as sodium hydride provides the olefin 5. Reduction of the ketone group with a reducing agent such as sodium borohydride provides the alcohol 6. The alcohol 6 may subsequently be protected with a suitable protecting group such as t-butyldimethylsilylchloride and thence, if desired, the protected amide (such as paramethoxybenzyl = R) it would be deprotected utilizing an oxidizing agent such as ceric ammonium nitrate. The free amide then be reacted with a strong base (sodium hydride) and then reacted with a suitably elaborated alkyl halide RX to provide the final products.

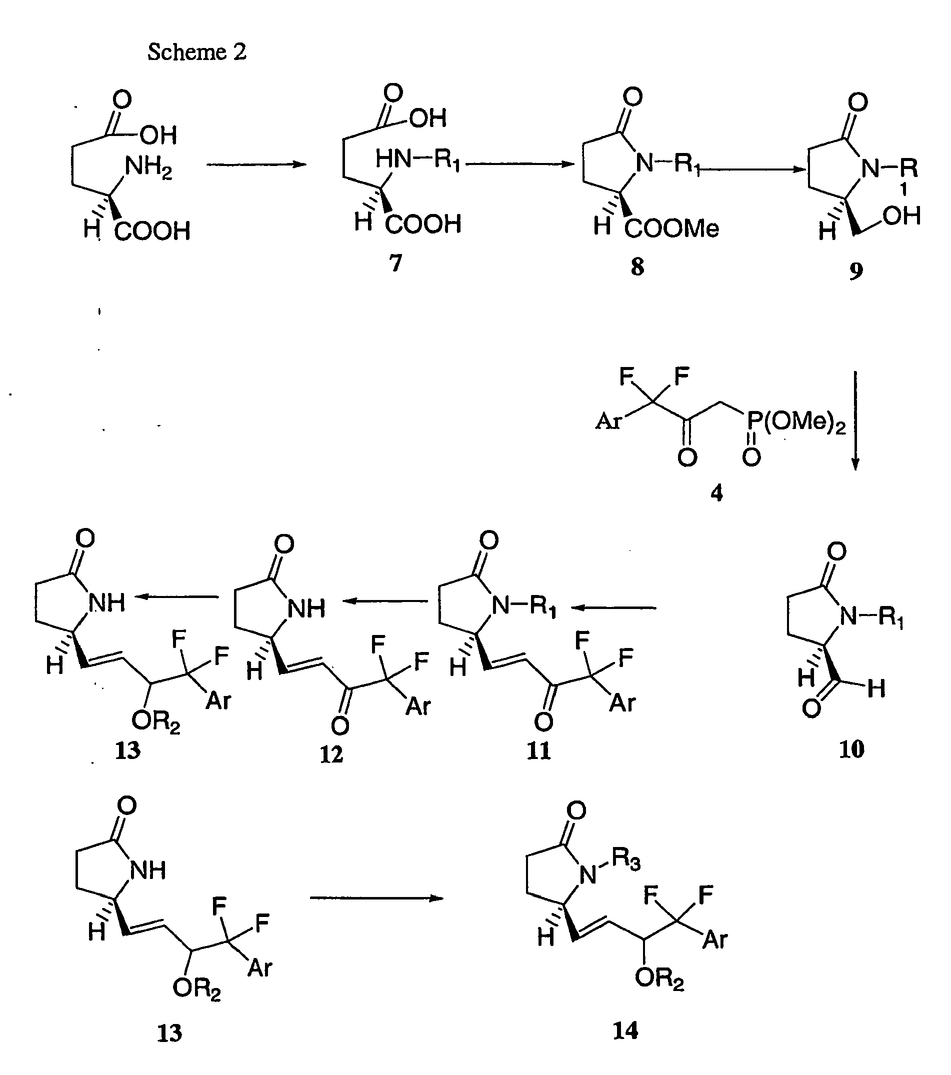

An alternative scheme is shown in Scheme 2. Glutamic acid is treated with an aromatic aldehyde such as paramethoxybenzyldehyde, in the presence of reducing agents such as sodium borohydride or sodium cyanoborohydride to provide the N-alkylated product 7. Cyclization and ester synthesis are accomplished using methanol and acidic catalysts to afford 8. Reduction of the ester to the alcohol 9 is accomplished using a reducing agent such as sodium borohydride and then oxidation of the alcohol moiety with common oxidation reagents such as pyridine, sulfur trioxide, etc. provides the aldehyde 10. Reaction of the aldehyde 10 with difluoroketophosphonate 4 in the presence of a strong base such as LDA or sodium hydride allows the formation of enone 11. The protecting group R₁ can then be removed in an oxidation step using a reagent such as cerium ammonium nitrate to afford the lactam 12. The enone moiety is reduced to the allylalcohol using a common reducing agent such as sodium borohydride and then protected in the usual manner by reaction with t-butyldimethylsilylchloride to afford a protected alcohol 13. The amide 13 can then be alkylated with a variety of groups R₃ by reaction first with a strong base such as sodium hydride in a polar non protic solvent in the presence of a phase-transfer catalyst such as tetrabutylammoniumiodide and then addition of electrophilic reagent R₃X where X is the halogen or a suitable leaving group to provide 14.

If R₃ is an aromatic group, the reaction conditions are that compound 13 is mixed with a base such as cesium carbonate in dioxane, the aromatic R₃X where X is halogen or triflate and a catalytic amount of palladium catalyst and the mixture is heated.

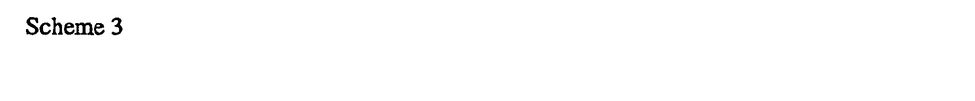

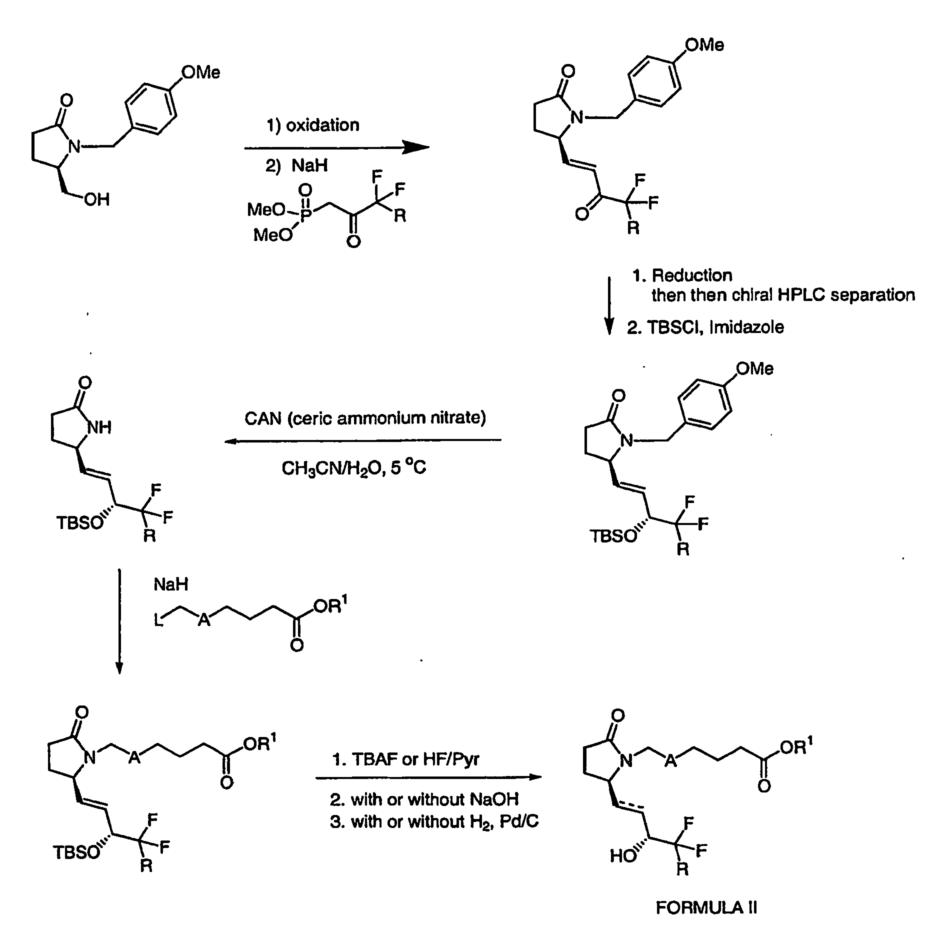

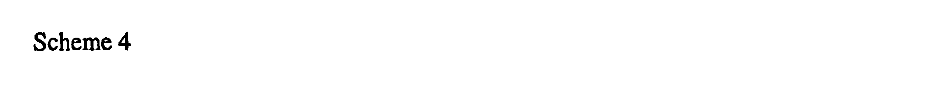

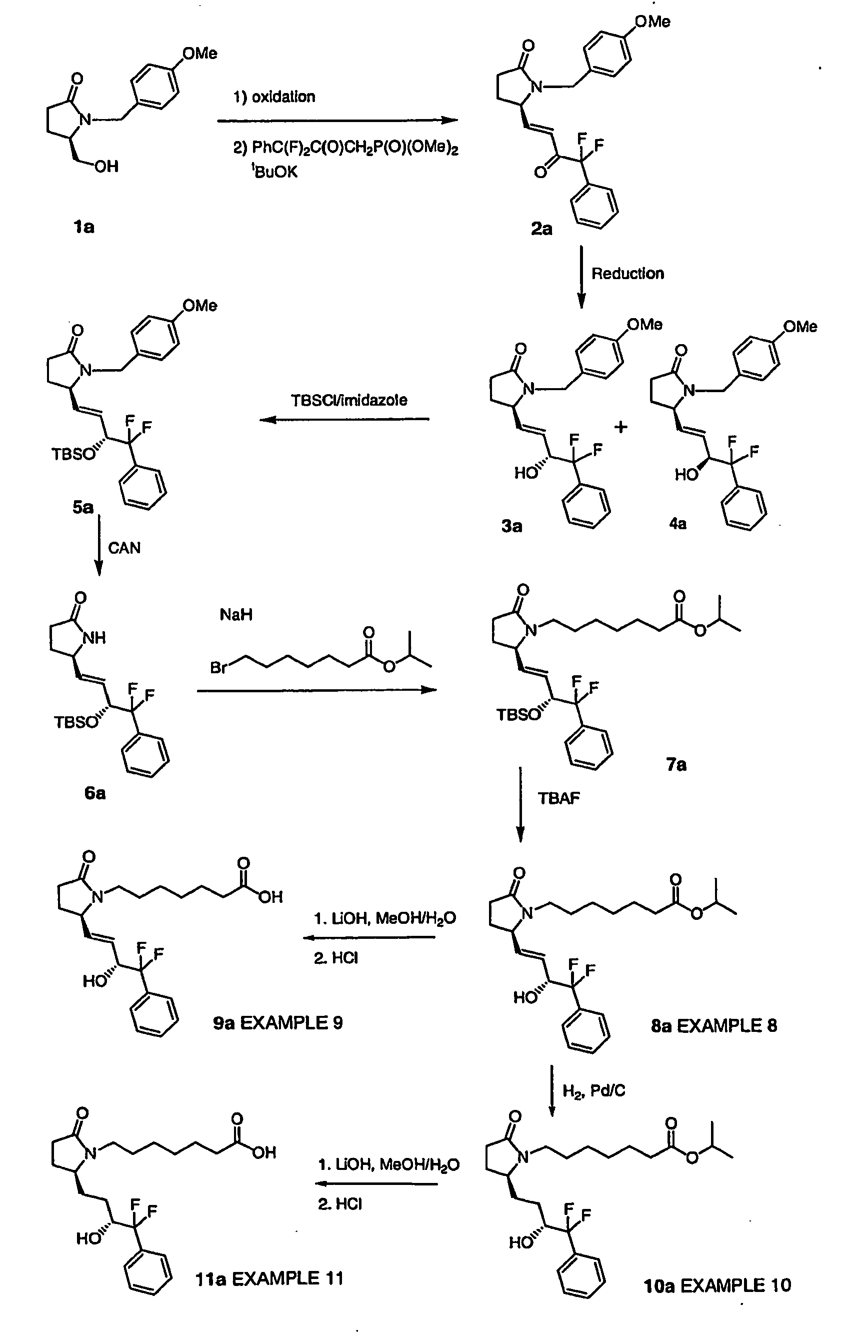

### EXAMPLES

### Preparation 1

### 7-(2R-formyll-5-oxo-pyrrolidin-1-yl)-heptanenitrile

### Step A: 7-[2R-(tert-Butyl-dimethyl-silanyloxymethyl)-5-oxo-pyrrolidin-1-yl]-heptanenitrile.

To a mixture of NaH (60% in oil, 3.836 g, 0.0959 mol, washed with 25 mL DMF in DMF (250 mL) was added a solution of 5R-(tert-butyl-dimethyl silanyloxymethyl)-pyrrolidin-2-one (Tetrahdedron: Asymmetry, 1996, 7, 2113) (20.00 g, 87.19 mmol) in DMF (50 mL). The reaction was stirred at room temperature for 1.5 h and a solution of 7-bromoheptanonitrile (16.574 g, 87.19 mmol) in DMF (50 mL) was added. The reaction was stirred at 90°C for 3 h. The reaction was cooled to 10 room temperature and water (750 mL) was added. The aqueous solution was washed with EtOAc (4x250 mL). The combined organic solutions were washed with water (2x250 mL), dried (MgSO4), filtered, and concentrated. Purification by medium pressure chromatography eluting with a solvent gradient (9:1 hexanes:EtOAc to 7:3 hexanes:EtOAo to 1:1 hexanes:EtOAc) provided 7-[2R-(tert-butyl-dimethyl 15 silanyloxymethyl)-5-oxo-pyrrolidin-1-yi)-heptanenitrile (22.46 g). 1H NMR (CDCl₃) δ 3.69-3.55 (m, 4H), 2.99 (m, 1 H), 2.42 (m, 1 H), 2.34-2.24 (m, 3H), 2.05 (m, 1 H), 1.81 (m, 1 H), 1.67-1.42 (m, 6H), 1.31 (m, 2H), 0.86 (s, 9H), 0.03 (s, 6H); MS 339.3 (M+1).

### Step B: 7-(2R-Hydroxymethyl-5-oxo-pyrrolidin-1-yl-heptanenitrile.

A solution of tetrabutylammonium fluoride (1M in THF, 100.0 mL, 100.0 mmol) was slowly added to a solution of 7-[2R-(tert-butyl-dimethyl-silanyloxymethyl)-5-oxo-pyrrolidin-1-yl]- heptanenitrile (22.39 g, 66.13 mmol) in THF (400 mL) at 0°C. The reaction was warmed to room temperature and was stirred for 4 h. Saturated aqueous NaHCO₃ (250 mL) was added and the volatiles were removed in vacuo. The remaining aqueous solution was washed with CHCl₃ (4x2OO mL). The combined organic solutions were dried (MgSO₄), filtered, and concentrated. Purification by medium pressure chromatography eluting with a solvent gradient (9:1 hexanes:EtOAc to 4:1 hexanes:EtOAc to 7:3 hexanes:EtOAc to 6:4 hexanes:EtOAc to 1:1 hexanes:EtOAc to EtOAc to 9:1 EtOAc:MeOH) provided 7-(2R-hydroxymethyl-5-oxo-pyrrolidin-1 -yl)-heptanenitrile (14.922 g). 1 H NMR (CDCl₃) δ 3.78 (dd, 1 H), 3.71-3.58 (m, 3H), 3.00 (m, 1H), 2.46 (m, 1 H), 2.36-2.27 (m, 3H), 2.08 (m, 1 H), 1.93 (m, 1 H), 1.77 (m, 1 H), 1.68-1.43 (m, 6H), 1.32 (m, 2H); MS 225.1 (M+1).

### Step C:

### 7-(2R-formyl-5-oxo-pyrrolidin-1-yl-heptanenitrile.

To a solution of 7-(2R-hydroxymethyl-5-oxo-pyrrolidin-1 -yl)-heptanenitrile (336 mg, 1.5 mmole) in CH₂Cl₂ (7 ml) was added portion-wise Dess-Martin periodinane (636 mg, 1.5 mmole) and the reaction mixture was stirred 1h at rt. Solvent is removed in vacuo, and the residue is triturated with toluene, filtered on celite and solvent removed to give 7-(2R-formyl-5-oxo-pyrrolidin-1-yl)-heptanenitrile as an oil.

### Preparation 2

### Dimethyl 3,3-difluoro-2-oxo-3-phenyl-propylphosphonate

To a solution of dimethyl methanephosphonate (1.139 g, 9.18 mmole) in 20 ml THF is added dropwise nBuLi (1.6 M in hexanes, 5.73 ml, 9.18 mmole) at - 78°C. This solution is stirred 30 min at - 78°C and then added to a solution of 2,2-difluorophenylacetic acid methyl ester (1.75 g, 8.74 mmole) at - 78°C. The reaction mixture is allowed to reach rt and then acetic acid (1.5 ml) and 10 ml water are added. The aqueous phase is extracted three times with 30 ml AcOEt, the organic phases are then washed with water, brine, dried on Na₂SO₄ and the solvent is removed under reduced pressure. Purification of the residual oil by silica gel flash chromatography (3:7 Acetone : toluene) to give Dimethyl 3,3-difluoro-2-oxo-3-phenyl-propylphosphonate as an oil. 1 H NMR (CDCl₃) δ 7.65-7.40 (m, 5 H), 3.71-3.58 (m, 3H), 3.77 (s, 3 H), 3.74 (s, 3 H), 3.35 (d, *J =* 22 Hz, 2H); MS 279.1 (M+1).

### Preparation 3

### (5R)-1-(4-chlorobutyl)-5-[(1E)-3-hydroxy-4-phenylbut-1-enyl]-pyrrolidin-2-one

### Step A: (5R)-(tert-butyl-dimethyl-silanyloxymethyl)-1-(4-chlorobutyl)pyrrolidin-2-one

To a solution of (5*R*)-(tert-butyl-dimethyl-silanyloxymethyl)-pyrrolidin-2-one (Tetrahedron: Asymmetry, 1996, 7, 2113) (2.83 g, 12.34 mmol) in 60 ml DMF was added NaH (95%, 325.7 mg, 13.57 mmol) in one portion and the mixture was heated at 50°C for 30 min. Then 4-bromo-1-chlorobutane (2.96 g, 17.27 mmol) and a catalytic amount of nBu₄NI were added and the mixture was stirred at 50°C for 1 h. The reaction was cooled to room temperature and water (100 ml) was added. The aqueous phase was extracted with AcOEt (4x200ml), the organic phases were washed with water (200 ml), brine (100 ml), dried on MgSO₄, filtered and the solvent was removed under reduced pressure. The residual oil was purified by flash column-chromatography on silica gel (eluent AcOEt 1: Hexanes 1) to provide (5*R*)-(tert-butyl-dimethyl-silanyloxymethyl)-1-(4-chlorobutyl)pyrrolidin-2-one as an oil. ¹H NMR (CDCl₃) 3.71-3.53 (m, 6H), 3.05 (m, 1H), 2.46-2.24 (m, 2H), 2.05 (m, 1H), 1.84-1.61 (m, 4H), 0.85 (s, 9H), 0.03 (s, 6H); MS 320.2-322.2 (M+1).

### Step B: (5R)-1-(4-chlorobutyl)-5-(hydroxymethyl)pyrrolidin-2-one

To a solution of (5*R*)-(tert-butyl-dimethyl-silanyloxymethyl)-1-(4-chlorobutyl)pyrrolidin-2-one (1.95 g, 6.11 mmol) in CH₂Cl₂ (25 ml) in a Teflon Erlenmeyer at 0°C was added dropwise HF-pyridine complex (1 ml), and the solution was allowed to reach room temperature, and was stirred for 1.5 h. Water (20 ml) and 1N HCl (1 ml) were added to the reaction mixture. The aqueous phase was extracted with CH₂Cl₂ (4x30ml), the organic phases was washed with brine (20 ml), dried on MgSO₄, filtered and the solvent was removed under reduced pressure. The residual oil was purified by flash column-chromatography on silica gel (eluent Acetone 1: Toluene 1) to provide (5*R*)-1-(4-chlorobutyl)-5-(hydroxymethyl)pyrrolidin-2-one as an oil. ¹H NMR (CDCl₃) 4.00 (s, 1H), 3.71-3.53 (m, 6H), 3.03 (m, 1H), 2.46-2.22 (m, 2H), 2.14-1.88 (m, 2H), 1.79-1.55 (m, 4H); MS 206.1-208.1 (M+1).

### Step C: (2R)-1-(4-chlorobutyl)-5-oxopyrrolidine-2-carboxaldehyde

To a solution of (5*R*)-1-(4-chlorobutyl)-5-(hydroxymethyl)pyrrolidin-2-one (309.6 mg, 1.5 mmol) in CH₂Cl₂ (7 ml) was added Dess-Martin periodinane (638 mg, 1.5 mmol) portionwise over 40 min at room temperature. After 1 h, the solvent was removed under reduced pressure, and the residue triturated with Et₂O (3x5 ml), filtered on a celite plug, and the solvent removed. (2*R*)-1-(4-chlorobutyl)-5-oxopyrrolidine-2-carboxaldehyde was obtained as a colorless oil. ¹H NMR (CDCl₃) 9.58 (s, 1H), 4.18 (m, 1H), 3.65 (m, 1H), 3.53 (t, *J*= 8 Hz, 2H), 3.08 (m, 1H), 2.43 (m, 2H), 2.30(m, 1H), 2.08 (m, 1H), 1.78-1.56 (m, 4H).

### Step D: (5R)-1-(4-chlorobutyl)-5-[(1E)-3-oxo-4-phenylbut-1-enyl]pyrrolidin-2-one

To a solution of (3-phenyl-2-oxo-propyl)-phosphonic acid dimethyl ester (938 mg, 4 mmol) in DME (20 ml) at 0°C was added portionwise NaH 95 % (100.8 mg, 4.2 mmol), and the mixture was stirred 20 min at 0°C. Then a solution of (2*R*)-1-(4-chlorobutyl)-5-oxopyrrolidine-2-carboxaldehyde in DME (5 ml) was added dropwise and the reaction mixture was allowed to reach room temperature, and stirred overnight. A half-saturated solution of NH₄Cl (10 ml) was added and the aqueous phase was extracted with AcOEt (4x60ml); the organic phases was washed with water (20 ml), brine (20 ml), dried on MgSO₄, filtered and the solvent was removed under reduced pressure. The residual oil was purified by flash column-chromatography on silica gel (eluent Acetone 2: Toluene 8) to provide (5*R*)-1-(4-chlorobutyl)-5-[(1*E*)-3-oxo-4-phenylbut-1-enyl]pyrrolidin-2-one as an oil. ¹H NMR (CDCl₃) 7.35-7.20 (m, 5H), 6.64 (dd, *J* = 15.7 Hz, 8.2 Hz, 1H), 6.25 (d, *J* = 15.7 Hz, 1H), 4.17 (m, 1H), 3.85 (s, 2H), 3.55-3.50 (m, 3H), 2.77 (m, 1H), 2.43-2.17 (m, 3H), 1.81-1.75 (m, 1H), 1.70-1.51 (m, 4H).

### Step E: (5R)-1-(4-chlorobutyl)-5-[(1E)-3-hydroxy-4-phenylbut-1-enyl]pyrrolidin-2-one

To a solution of (5*R*)-1-(4-chlorobutyl)-5-[(1*E*)-3-oxo-4-phenylbut-1-enyl]pyrrolidin-2-one (161 mg, 0.50 mmol) in MeOH (5 ml) at -20°C was added portionwise NaBH₄ (31 mg, 0.8 mmol). The mixture was stirred at -20°C for 1 h, and the solvent was removed under reduced pressure. The residue was dissolved in a mixture of water (5 ml) and 1N HCl (1 ml), the aqueous phase was extracted with AcOEt (3x15ml); the organic phases was washed with water (5 ml), brine (5 ml), dried on MgSO₄, filtered and the solvent was removed under reduced pressure. The residual oil was purified by flash column-chromatography on silica gel (eluent Acetone 4: Toluene 6) to provide both diastereoisomers of (5*R*)-1-(4-chlorobutyl)-5-[(1*E*)-3-hydroxy-4-phenylbut-1-enyl]pyrrolidin-2-one as an oil. ¹NMR (CDCl₃) 7.36-7.22 (m, 5H), 5.78 (m, 1H), 5.51 (m, 1H), 4.44 (m,1H), 4.07 (m, 1H), 3.59-3.45 (m, 3H), 2.95-2.77 (m, 3H), 2.44-2.19 (m, 3H), 2.43-2.17 (m, 3H), 1.70-1.55 (m, 5H).

### Preparation 4

### (5R)-5-[(1E)-3-hydroxy-4-phenylbut-1-enyl]-1-{4-[(triisopropylsilyl)thio]butyl}-pyrrolidin-2-one

To a solution of (5*R*)-1-(4-chlorobutyl)-5-[(1*E*)-3-hydroxy-4-phenylbut-1-enyl]pyrrolidin-2-one (273.9 mg, 0.852 mmol) in THF (5 ml) were added triisopropylsilylsulfide (324.4 mg, 1.70 mmol), a catalytic amount of nBu₄NI and portionwise NaH 95 % (30.7 mg, 1.28 mmol). The mixture was heated to 50°C for 1 h. The reaction was cooled to room temperature and water (2 ml) was added. The aqueous phase was extracted with AcOEt (4x10ml), the organic phases were washed with water (2 ml), brine (2 ml), dried on MgSO₄, filtered and the solvent was removed under reduced pressure. The residual oil was purified by flash column-chromatography on silica gel (eluent AcOEt 1: Hexanes 3) to provide both diastereoisomers of (5*R*)-5-[(1*E*)-3-hydroxy-4-phenylbut-1-enyl]-1-14-[(triisopropylsilyl)thio]butyl}pyrrolidin-2-one as an oil. ¹H NMR (CDCl₃) 7.30-7.16 (m, 5H), 5.72 (m, 1H), 5.45 (m, 1H), 4.37 (m, 1H), 4.02 (m, 1H), 3.44 (m, 1H), 2.86-2.79 (m, 3H), 2.51 (m, 2H), 2.35-2.12 (m, 3H), 1.94 (s, 1H), 1.64-1.50 (m, 5H), 1.2 (m, 3H), 1.05 (d, J = 8.0 Hz, 18H); MS 476.4 (M+1).

The difluoro component in the examples below, unless otherwise indicted, can be added to the following examples in accordance with schemes 1 and 2 herein.

### EXAMPLE 1

### (5R)-5-[(1E)-3-hydroxy-difluoro-4-phenylbut-1-enyl]-1-{4-[(1-methyl-1H-tetrazol-5-yl)thio]butyl}pyrrolidin-2-one

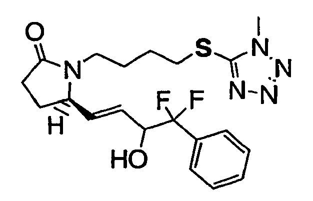

To a solution of the (5*R*)-1-(4-chlorobutyl)-5-[(1*E*)-3-hydroxy-4-phenylbut-1-enyl]pyirolidin-2-one (50.0 mg, 0.155 mmol) in DMF (0.5 ml) wae added 5-mercapto-1-methyltetrazole sodium salt, and a catalytic amount of nBu₄NI. The mixture is heated to 50°C overnight. The reaction is cooled to room temperature and water (5 ml) is added. The aqueous phase is extracted with AcOEt (4x10ml), the organic phases are washed with water (2 ml), brine (2 ml), dried on MgSO₄, filtered and the solvent is removed under reduced pressure. The residual oil is purified by flash column-chromatography on silica gel (eluent AcOEt 2: Hexanes 3) to provide both diastereoisomers of (5*R*)-5-[(1*E*)-3-hydroxy-4-difluoro-4-phenylbut-1-enyl]-1-{4-[(1-methyl-1*H*-tetrazol-5-yl)thio]butyl}pyrrolidin-2-one.

### EXAMPLE 2 (reference)

### 4-{(2R)-2-[(1E)-3-hydroxy-4-difluoro-4-phenylbut-1-enyl]-5-oxopyrrolidin-1-yl}butyl thiocyanate

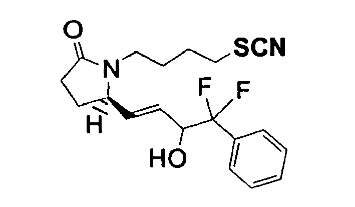

To a solution of the (5*R*)-1-(4-chlorobutyl)-5-[(1*E*)-3-hydroxy-4-phenylbut-1-enyl]pyrrolidin-2-one (50.0 mg, 0.155 mmol) in DMF (1 ml) are added potassium thiocyanate (150.7 mg, 1.55 mmol), and a catalytic amount of nBu₄NI. The mixture is heated to 50°C overnight. The reaction is cooled to room temperature and water (5 ml) is added. The aqueous phase is extracted with AcOEt (4x10ml), the organic phases are washed with water (2 ml), brine (2 ml), dried on MgSO₄, filtered and the solvent is removed under reduced pressure. The residual oil is purified by flash column-chromatography on silica gel (eluent Acetone 4: Toluene 6) to provide both diastereoisomers of 4-{(2*R*)-2-[(1*E*)-3-hydroxy-4-difluoro-4-phenylbut-1-enyl]-5-oxopyrrolidin-1-yl} butyl thiocyanate.

### EXAMPLE 3

### 3-[4-{(2R)-2-[(1E)-3-hydroxy-4-difluoro-4-phenylbut-1-enyl]-5-oxopyrrolidin-1-yl}butyl)thio]propanoic acid

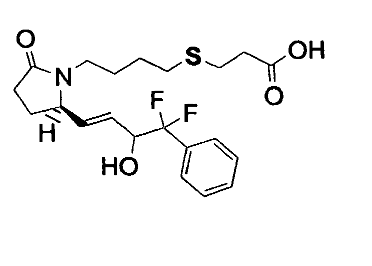

### Step A : methyl 3-[4-{(2R)-2-[(1E)-3-hydroxy-4-phenylbut-1-enyl]-5-oxopyrrolidin-1-yl}butyl)thio]propanoate

To a solution of the (5*R*)-1-(4-chlorobutyl)-5-[(1*E*)-3-hydroxy-4-phenylbut-1-enyl]pyrrolidine-2-one (50.0 mg, 0.155 mmol) in DMF (0.8 ml) are added 3-mercaptopropanoic acid methyl ester (93.0 mg, 0.755 mmol), a catalytic amount of nBu₄NI and then dropwise 1M MeONa (0.62 ml, 0.62mmol). The mixture is heated to 80°C for 24h. The reaction is cooled to room temperature and water (6 ml) is added. The aqueous phase is extracted with AcOEt (4x10ml), the organic phases are washed with water (2 ml), brine (2 ml), dried on MgSO₄, filtered and the solvent is removed under reduced pressure. The residual oil is purified by flash column-chromatography on silica gel (eluent Acetone 4: Toluene 6) to provide both diastereoisomers of methyl 3-[4-{(2*R*)-2-[(1*E*)-3-hydroxy-4-phenylbut-1-enyl]-5-oxopyrrolidin-1-yl}butyl)thio]propanoate.

### Step B: 3-[4-{(2R)-2-[(1E)-3-hydroxy-4-phenylbut-1-enyl]-5-oxopyrrolidin-1-yl}butyl)thio]propanoic acid

To a solution of methyl 3-[4-{(2*R*)-2-[(1*E*)-3-hydroxy-4-phenylbut-1-enyl]-5-oxopyrrolidin-1-yl}butyl)thio]propanoate (19.6 mg, 0.0484 mmol) in MeOH/THF (1:1)(2 ml) is added a solution of 1N LiOH (0.051 ml, 0.051 mmol) at 0°C. The reaction mixture is stirred overnight at room temperature. 0.5N HCl (4 ml) is added, the aqueous phase is extracted with CH₂Cl₂ (4x10ml), the organic phases are washed with brine (2 ml), dried on MgSO₄, filtered and the solvent is removed under reduced pressure. The residual oil is purified by flash column-chromatography on silica gel (gradient CH₂Cl₂: MeOH: AcOH (100:0:0) to (95:5:0.5)) to provide both diastereoisomers of 3-[4-{(2*R*)-2-[(1*E*)-3-hydroxy-4-difluoro-4-phenylbut-1-enyl]-5-oxopyrrolidin-1-yl}butyl)thio]propanoic acid.

### EXAMPLE 4 (reference)

### [4-{(2R)-2-[(1E)-3-hydroxy-4-difluoro-4-phenylbut-1-enyl]-5-oxopyrrolidin-1-yl}butyl)thio]methanesulfonic acid

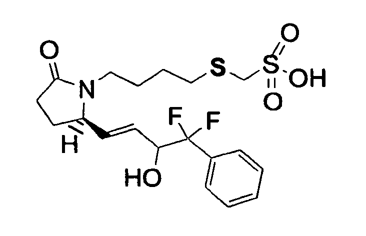

To a solution of the (5*R*)-5-[(1*E*)-3-hydroxy-4-phenylbut-1-enyl]-1-{4-[(triisopropylsilyl)thio]butyl}pyrrolidin-2-one (39.3 mg, 0.083 mmol) in THF (1 ml) are added sodium bromomethanesulfonate (32.6 mg, 0.165 mmol) and then dropwise 1M nBu₄NF (0.25 ml, 0.25mmol). The mixture was heated to 50°C for 1h. The reaction is cooled to room temperature and 1N HCl (2 ml) is added. The aqueous phase is extracted with Et₂O (4x10ml), the organic phases are washed with 1N HCl (2 ml), brine (2 ml), dried on Na₂SO₄, filtered and the solvent is removed under reduced pressure. The residual oil is purified by flash column-chromatography on silica gel (eluent CH₂Cl₂ 95: MeOH 5:AcOH 0.5) to provide both diastereoisomers of methyl [4-{(2*R*)-2-[(1*E*)-3-hydroxy-4-difluoro-4-phenylbut-1-enyl]-5-oxopyrrolidin-1-yl}butyl)thio]methanesulfonic acid.

### EXAMPLE 5 (reference)

### (5R)-5-[(1E)-3-hydroxy-4-difluoro-4-phenylbut-1-enyl]-1-[4-(methylsulfonyl)butyl]-pyrrolidin-2-one

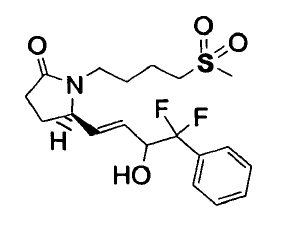

### Step A: (5R)-5-[(1E)-3-hydroxy-4-phenylbut-1-enyl[4-(methylthio)butyl]pyrrolidin-2-one

To a solution of the (5*R*)-5-[(1*E*)-3-hydroxy-4-phenylbut-1-enyl]-1-{4-[(triisopropylsilyl)thio]butyl}pyrrolidin-2-one (46.2 mg, 0.097 mmol) in THF (1 ml) were added methyliodide (17.6 mg, 0.126 mmol) and then dropwise 1M nBu₄NF (0.116 ml, 0.116mmol) at -78°C. The mixture is then stirred at room temperature for 1h. NH4Cl half saturated (2 ml) is added. The aqueous phase is extracted with AcOEt (5x8 ml), the organic phases are washed with brine (2 ml), dried on Na₂SO₄, filtered and the solvent is removed under reduced pressure. The residual oil is purified by flash column-chromatography on silica gel (eluent Acetone 4: Toluene 60) to provide (5*R*)-5-[(1*E*)-3-hydroxy-4-difluoro-4-phenylbut-1-enyl]-1-[4-(methylthio)butyl]-pyrrolidin-2-one.

### Step B: (5R)-5-[(1E)-3-hydroxy-4-phenylbut-1-enyl[4-(methylsulfonyl)butyl]-pyrrolidin-2-one

To a solution of (5*R*)-5-[(1*E*)-3-hydroxy-4-phenylbut-1-enyl]-1-[4-(methylthio)butyl]pyrrolidin-2-one (31.2 mg, 0.093 mmol) in CH₂Cl₂ : MeOH:H₂O (7:2:1)(5 ml) was added portionwise Oxone^{®} (172.9 mg, 0.281 mmol) at 0°C for 10 min., and 4 h at room temperature. 5% solution of NaHSO₃ (2 ml) is added. The aqueous phase is extracted with CH₂Cl₂ (4x10 ml), the organic phases are washed with water (5 ml), brine (2 ml), dried on MgSO₄, filtered and the solvent is removed under reduced pressure. The residual oil is purified by flash column-chromatography on silica gel (eluent Acetone 7: Toluene 30) to provide (5*R*)-5-[(1*E*)-3-hydroxy-4-difluoro- 4-phenylbut-1-enyl]-1-[4-(methylsulfonyl)butyl]pyrrolidin-2-one.

### EXAMPLE 6

### [4-{(2R)-2-[(1E)-3-hydroxy-4-difluoro-4-phenylbut-1-enyl]-5-oxopyrrolidin-1-yl}butyl)thio]acetic acid

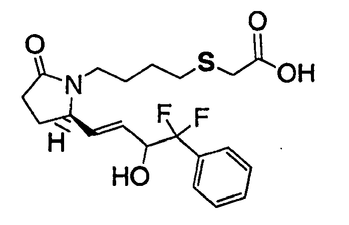

### Step A: methyl ({[4-{(2R)-2-({[tert-butyl(dimethyl)silyl]oxy}methyl)-5-o xopyrolidin-1-yl]butyl}thio)acetate

To a solution of (5*R*)-(tert-butyl-dimethyl-silanyloxymethyl)-pyrrolidin-2-one (Tetrahedron: Asymmetry,1996, 7, 2113) (1.5 g, 6.55 mmol) in 30 ml DMF is added NaH 95% (173.0 mg, 7.20 mmol) in one portion and the mixture is heated at 50°C for 30 min. Then 4-bromo-1-chlorobutane (1.347 g, 7.86 mmol) and a catalytic amount of nBu₄NI are added and the mixture is stirred at 50 °C for 1 h. The reaction is cooled to room temperature and methyl thioglycolate (1.39 g, 13.1 mmol), then dropwise addition of 4.9N MeONa (2.4 ml, 11.79 mmol). The mixture is stirred overnight at room temperature and water (150 ml) is added. The aqueous phase is extracted with AcOEt (4x150ml), the organic phases are washed with water (200 ml), brine (100 ml), dried on MgSO₄, filtered and the solvent is removed under reduced pressure. The residual oil is purified by flash column-chromatography on silica gel (eluent AcOEt 1: Hexanes 1) to provide methyl ({[4-{(2*R*)-2-({[tert-butyl(dimethyl)silyl]oxy}methyl)-5-oxopyrrolidin-1-yl]butyl}thio)acetate.

### Step B: methyl ({4-[(2R)-2-(hydroxymethyl)-5-oxopyrrolidin-1-yl]butyl}thio)acetate

To a solution of methyl ({[4-{(2*R*)-2-({[tert-butyl(dimethyl)silyl]oxy}methyl)-5-oxopyrrolidin-1-yl]butyl}thio)acetate (571 mg, 1.47 mmol) in CH₂Cl₂ (8 ml) in a Teflon Erlenmeyer at 0°C is added dropwise HF-pyridine complex (0.8 ml), and the solution is allowed to reach room temperature, and is stirred for 1.5 h.. Water (20 ml) and 1N HCl (1 ml) were added to the reaction mixture. The aqueous phase is extracted with CH₂Cl₂ (4x30ml), the organic phases is washed with brine (20 ml), dried on MgSO₄, filtered and the solvent is removed under reduced pressure to provide methyl ({4-[(2*R*)-2-(hydroxymethyl)-5-oxopyrrolidin-1-yl]butyl}thio)acetate.

### Step C: Methyl ({4-[(2R)-2-formyl-5-oxopyrrolidin-1-yl]butyl}thio)acetate

To a solution of methyl ({4-[(2*R*)-2-(hydroxymethyl)-5-oxopyrrolidin-1-yl]butyl}thio)acetate (634.5 mg, 2.30 mmol) in CH₂Cl₂ (15 ml) is added Dess-Martin periodinane (975 mg, 1.5 mmol) portionwise over 40 min at room temperature. After 1 h, the solvent is removed under reduced pressure, and the residue triturated with Et₂O (3x5 ml), filtered on a Celite plug, and the solvent removed. Methyl ({4-[(2*R*)-2-formyl-5-oxopyrrolidin-1-yl]butyl}thio)acetate is obtained.

### Step D: methyl [(4-{(5R)-2-oxo-5-[(1E)-3-oxo-4-phenylbut-1-enyl]pyrrolidin-1-yl}butyl)thio]acetate

To a solution of (3-phenyl-2-oxo-propyl)-phosphonic acid dimethyl ester (264 mg, 1.09 mmol) in DME (5 ml) at 0°C is added portionwise NaH 95 % (26 mg, 1.09 mmol), and the mixture is stirred 20 min at 0°C. Then a solution of methyl ({4-[(2*R*)-2-formyl-5-oxopyrrolidin-1-yl]butyl}thio) (270 mg, 0.99 mmol) in DME (2 ml) is added dropwise and the reaction mixture is allowed to reach room temperature, and stirred overnight. A half-saturated solution of NH₄Cl (5 ml) is added and the aqueous phase is extracted with AcOEt (4x10ml); the organic phases is washed with water (10 ml), brine (10 ml), dried on MgSO₄, filtered and the solvent is removed under reduced pressure. The residual oil is purified by flash column-chromatography on silica gel (eluent AcOEt) to provide methyl [(4-{(5*R*)-2-oxo-5-[(1*E*)-3-oxo-4-phenylbut-1-enyl]pyrrolidin-1-yl}butyl)thio]acetate.

### Step E: methyl [(4-{(2R)-2-[(1E)-3-hydroxy-4-phenylbut-1-enyl]-5-pyrrolidin-1-yl}butyl)thio]acetate

To a solution of methyl [(4-{(5*R*)-2-oxo-5-[(1*E*)-3-oxo-4-phenylbut-1-enyl]pyrrolidin-1-yl}butyl)thio]acetate (295.6 mg, 0.75 mmol) in MeOH (5 ml) at-20°C was added portionwise NaBH₄ (27.6 mg, 1.2 mmol). The mixture is stirred at - 20°C for 1 h, and the solvent is removed under reduced pressure. The residue is dissolved in a mixture of water (5 ml) and 1N HCl (1 ml), the aqueous phase is extracted with AcOEt (3x15ml); the organic phases is washed with water (5 ml), brine (5 ml), dried on MgSO₄, filtered and the solvent is removed under reduced pressure. The residual oil is purified by flash column-chromatography on silica gel (eluent Acetone 4: Toluene 6) to provide both diastereoisomers of methyl [(4-{(2*R*)-2-[(1*E*)-3-hydroxy-4-phenylbut-1-enyl]-5-pyrrolidin-1-yl}butyl)thio]acetate.

### Step F: [4-{(2R)-2-[(1E)-3-hydroxy-4-phenylbut-1-enyl]-5-oxopyrrolidin-1-yl}butyl)thio]acetic acid

To a solution of methyl [(4-{(2*R*)-2-[(1*E*)-3-hydroxy-4-phenylbut-1-enyl]-5-pyrrolidin-1-yl}butyl)thio]acetate (90.0 mg, 0.23 mmol) in MeOH/THF (1:2)(5 ml) is added a solution of 1N LiOH (0.46 ml, 0.46 mmol) at 0°C. The reaction mixture is stirred 4 h at room temperature. 1N HCl (3 ml) is added, the aqueous phase is extracted with CH₂Cl₂ (4x10ml), the organic phases are washed with brine (2 ml), dried on MgSO₄, filtered and the solvent is removed under reduced pressure. The residual oil is purified by flash column-chromatography on silica gel (gradient CH₂Cl₂: MeOH: AcOH (100:0:0) to (94:6:0.5)) to provide both diastereoisomers of [4-{(2*R*)-2-[(1*E*)-3-hydroxy-4-difluoro-4-phenylbut-1-enyl]-5-oxopyrrolidin-1-yl}butyl)thio]-acetic acid.

### EXAMPLE 7

### (5R)-5[(1E)-4,4-difluoro-3-hydroxy-4-phenylbut-1-enyl]-1-[6-(1H-tetraazol-5-yl)hexyl]pyrrolidin-2-one

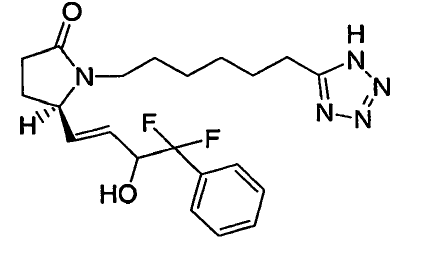

### Step A: 7-{2-Oxo-5R-[3-hydroxy-4,4-difluoro-4-phenyl)-but-1-enyl]-pyrrolidin-1-yl}-heptanenitrile.

To a solution of dimethyl 3,3-difluoro-2-oxo-3-phenyl-propylphosphonate (405 mg, 1.46 mmole) in THF (5 ml) is added NaH (36.8 mg,1.53 mmole) at 0°C, and the reaction mixture is stirred for 30 min at 0°C. A solution of 7-(2R-formyl-5-oxopyrrolidin-1 -yl)-heptanenitrile (325 mg, 1.46 mmole) in THF (5 ml) is the addes to the above mixture. After 30 min at 0°C, the mixture is allowed to reach rt for 2h.

Addition of a saturated solution of ammonium chloride (5 ml), extraction with AcOEt (3x15 ml); the organic phases are washed with brine, dried on Na₂SO₄ and the solvent is removed. The residue is purified by a silica gel flash chromatography (70 : 30 AcOEt : Hexanes) to give 7-{2-Oxo-5R-[3-oxo-4,4-difluoro-4- phenyl)-but-1-enyl]-pyrrolidin-1-yl}heptanenitrile as a oil. This oil is immediately dissolved in MeOH (5 ml), and CeCl₃ .7H₂O (223 mg, 0.6 mmole) is added at -20°C. NaBH₄ (36.5 mg, 0.96 mmole) is added portionwise, and the mixture is allowed to reach rt. HCl 1N (1 ml) is added, solvent is removed in vacuo. The residue is dissolved in water (5 ml), extracted with AcOEt (3x10 ml) and the organic phases are washed with brine, dried on Na₂SO₄ and solvent removed. Purification by flash silica gel chromatography (30:70 Acetone : Toluene) to give 7-{2-Oxo-5R-[3-hydroxy-4,4-difluoro-4-phenyl)-but-1-enyl]-pyrrolidin-1-yl}-heptanenitrile as a oil. 1 H NMR (CDCl₃) δ 7.48-7.3 (m, 5 H), 5.65-5.61 (m, 1), 5.57-5.53 (m, 1h), 4.5 (m, 1 H), 3.48 (m, 1H), 3.35 (m, 1H), 2.68 (m, 1H), 2.32-2.20 (m, 5H), 2.10 (m, 1H), 1.55-1.15 (m, 8H); MS 377.3 (M+1).

### Step B:

To 7-{2-Oxo-5R-[3-hydroxy-4,4-difluoro-4-phenyl)-but-1-enyl]-pyrrolidin-1-yl}-heptanenitrile (193.5 mg, 0.51 mmole) is added Tributylstannylazide (512 mg, 1.54 mmole) and the mixture is heated at 100°C for 8h. The reaction mixture is dissolved in AcOEt and worked-up with a mixture of 1:1 (1N HCl : 5% KF). The aqueous phase is extracted by AcOEt (3x10 ml), and the organic phases are washed with brine, dried on Na₂SO₄ and solvent removed. Purification by silica gel filtration, gradient (CH₂Cl₂: MeOH : 0.1% HCOOH) from (100 : 0) to (94 :6) to afford (5*R*)-5[(1*E*)-4,4-difluoro-3-hydroxy-4-phenylbut-1-enyl]-1-[6-(1H-tetraazol-5-yl)hexyl]pyrrolidin-2-one as an oil. 1 H NMR (CDCl₃) δ 7.5-7.35 (m, 5H), 5.7 (m, 1H), 5.6 (m, 1H), 4.6 (m, 1H), 4.05 (m, H), 3.9 (m, 2H), 2.75 (m, 1H), 2.4 (m, 2H), 2.2 (m, 1H), 1.80-1.6 (m, 3H), 1.4-1.05 (m, 6H); MS 420.3 (M+1), 418.3 (M-1).

### Examples 8-11 are prepared in accordance with Scheme 4.

### EXAMPLE 8

### isopropyl 7-{(2R)-2-[(1E,3R)-4,4-difluoro-3-hydroxy-4-phenylbut-1-enyl]-5-oxopyrrolidin-1-yl}heptanoate (8a)

### Step A

### (5R)-5-(hydroxymethyl)-1-(4-methoxybenzyl)pyrrolidin-2-one (1a)

The preparation of **1a** was carried out according to the literature procedure (see: Tetrahedron 1994, 6221)

### Step B

### (5R)-5-[(1E)-4,4-difluoro-3-oxo-4-phenylbut-1-enyl]-1-(4-methoxybenzyl)pyrrolidin-2-one (2a)

Oxalyl chloride (2.75 mL) was added dropwise to a solution of dimethylsulfoxide (2.45 mL) in CH₂Cl₂ (60 ml) at -72 °C under a stream of N₂, and the mixture was stirred 20 min at that temperature. A solution of (5*R*)-5-(hydroxymethyl)-1-(4-methoxybenzyl)pyrrolidin-2-one (**1a**, 6.74 g) in CH₂Cl₂ (30 ml) was then added via a cannula, and the mixture was stirred at -72 °C for 20 min. Triethylamine (8.45 ml) was then added and the mixture was stirred at -72 °C for an additional 30 min and then concentrated in vacuo (to remove most of the CH₂Cl₂). The residue was diluted with a mixture of ethyl acetate and diethyl ether and then filtered. The filtrated was concentrated to give the desired product. ¹H NMR (acetone-d₆) δ 9.50 (s, 1H), 7.20 (d, 2H), 6.90 (d, 2H), 4.80 (d, 1H), 4.14 (d, 1H), 4.06 (m, 1H), 3.79, (s, 3H), 2.20-2.45 (m, 3H), 2.12 (m, 1H).

To a solution of dimethyl 3,3-difluoro-2-oxo-3-phenylpropylphosphonate (United States Patent 4,320,136 March. 16, 1982) (2.076 g) in THF (17 mL) at 0 °C was added potassium tert-butoxide (963 mg) and the mixture was stirred for an additional 1 hour at 0 °C. To the mixture was then added (2*R*)-1-(4-methoxybenzyl)-5-oxopyrrolidine-2-carboxaldehyde in THF (10 mL) via cannula and the resultant mixture stirred at room temperature 2 hours and quenched with saturated NH₄Cl. The mixture was then extracted with ethyl acetate (3x) and the organic layer was washed with water, brine, dried over Mg₂SO₄, filtered and concentrated. The residue was purified by chromatography using 20% acetone/toluene as the eluent to give the desired product **2a**. ¹H NMR (400 MHz, CDCl₃): δ 7.60-7.45 (m, 5H), 7.00 (d, 2H), 6.78 (d, 2H), 6.45 (d, 1H), 4.90 (d, 1H), 4.10-4..00 (m, 1H), 3.80 (s, 3H), 3.70 (d, 1H), 3.35 (d, 1H), 2.55-2.40 (m, 2H), 2.25-2.15 (m, 1, 1H), 1.85-1.75 (m, 1H).

### Step C

### (5R)-5-{(1E,3R)-4,4-difluoro-3-hydroxy-4-phenylbut-1-enyl}-1-(4-methoxybenzyl)pyrrolidin-2-one (3a)

To a solution of **(2a)** (8.2 g, 21.2 mmol) in 80 mL CH₂Cl₂ was added (S)-CBS in toluene (10.6 mL, 10.6 mmol) and the mixture was cooled to -40 °C. A solution of catechol borane (6.8 mL, 63.8 mmol) in CH₂Cl₂ (20 mL) was added dropwiseand and the solution was stirred at -40 °C for one hour, and allowed to warm up to -20°C for two hours. The reaction mixture was quenched at -20 °C with 1 N HCl and was stirred for 4 hours at room temperature. The phases were separated and the organic phase was sequentially washed with 1N HCl, H₂O, 1 N NaOH, brine and dried over Na₂SO₄, filtered and concentrated in vacuo. The crude was purified by flash chromatography using 40-50% ethyl acetate/hexanes to give the desired products as a mixture of diastereomers as a pale yellow oil. MS (M + 1) 388.2. The mixture of diastereomers was further separated by preparative HPLC (ChiralPak AD^{®}). Eluting with 30% isopropyl alcohol in hexanes first gave isomer 4. Further elution afforded the major, more polar isomer **3a**.

### Step D

### (5R)-5-((1E,3R)-3-{[tert-butyl(dimethyl)silyl]oxy}-4,4-difluoro-4-phenylbut-1-enyl)-1-(4-methoxybenzyl)pyrrolidin-2-one (5a)

To a solution **3a** (365 mg) in DMF (3 mL) at room temperature was added imidazole (139 mg) followed by TBSCI (220 mg). The mixture was stirred over the weekend and then quenched with water. The mixture was extrated with ether (3x) and washed with water, brine, dried over Na₂SO₄, filtered and concentrated in vacuo. Purified by column chromatography (50% ethyl acetate : hexane) afforded compound **5a.** ¹H NMR (400 MHz, CDCl₃): δ 7.55-7.40 (m, 5H), 7.15-7.10 (m, 2H), 6.85-6.80 (m, 2H), 5.65-5.55 (m, 2H), 4.95 (2d, 1H), 4.55-4.45 (m, 1H), 3.90-3.85 (m, 1H), 3.80 (s, 3H), 3.60 (2d, 1H), 2.50-2.30 (m, 2H), 2.20-2.05 (m, 1H), 1.70-1.55 (m, 1H), 0.85 (d, 9H), 0.00 (t, 6H).

### Step E

### (5R)-5-((1E,3R)-3-{[tert-butyl(dimethyl)silyl]oxy}-4,4-difluoro-4-phenylbut-1-enyl)pyrrolidin-2-one (6a)

To a solution of **5a** (359 mg) in acetonitrile (20 mL) at 0 °C was added CAN (ceric(IV)ammonium nitrate) (2 g), water (2 mL) and the mixture was allowed to warm to room temperature for 4 hours. The mixture was extrated with ether (3x) and was washed with water, brine and dried over Na₂SO₄. Purification by column chromatography (50%-75%-100% ethyl acetate in hexane) afforded compound **5a**. ¹H NMR (400 MHz, CDCl₃): δ 7.50-7.40 (m, 5H), 5.70-5.65 (m, 2H), 4.50-4.42 (m, 1H), 4.20-4.13 (m, 1H), 2.37-2.30 (m, 3H), 1.80-1.70 (m, 1H), 0.87 (s, 9H), -0.05 (d, 6H).

### Step F

### isopropyl 7-{3-[(2R)-2-((1E,3R)-3-{[tert-butyl(dimethyl)silyl]oxy}-4,4-difluoro-4-phenylbut-1-enyl)-5-oxopyrrolidin-1-yl]heptanoate (7a)

To a solution of **5a** (314 mg) in DMF (dimethyl formamide - 5 mL) was added NaH 60% (36.4 mg) and the mixture was stirred at room temperature for 1h until gas evolution was ceased. Iso-propyl 7-bromoheptanoate (415 mg) and a crystal of NaI was added. The mixture was heated to 90 °C for 6h. After cooling to room temperature, the mixture was quenched with saturated NH₄Cl and extracted with diethyl ether (3 x). The organic layer was washed with water, brine and dried over Na₂SO₄. The crude was purified by flash chromatography. Eluting with 50-60% ethyl acetate in hexanes gave the desired product **6a.** ¹H NMR (400 MHz, acetone-d₆) δ 7.50 (m, 5H), 7.73, m, 2H), 4.96 (m, 1H), 4.70 (m, 1H), 4.18 (m, 1H), 3.44 (m, 1H), 2.74 (m, 1H), 2.30-2.18 (m, 5H), 1.70-1.20 (m, 9H), 1.20 (d, 6H), 0.87 (s, 9H), 0.05 (s, 3H) and -0.01 (s, 3H).

### Step G

### The title compound 8a

To a solution of **7a** (350 mg) in THF (tetrahydrofuran - 5 mL) was added TBAF (1M in THF, 1.3 mL) and the mixture was stirred at room temperature for 30 min. The solution was then diluted with ethyl acetate and washed with water (4x) and brine. After drying over Na2SO4, the organic layer was filtered and concentrated in vacuo to give the desired title compound **8a.** ¹H NMR (400 MHz, acetone-d₆) δ 7.56-7.47 (m, 5H), 5.79-5.65 (m, 2H), 5.05 (bs, 1H, OH), 4.95 (m, 1H), 4.66 (m, 1H), 4.12 (m, 1H), 3.30 (m, 1H), 2.70 (m, 1H), 2.30-2.15 (m, 5H), 1.70-1.20 (m, 9H), 1.20 (d, 6H).

### EXAMPLE 9

### 7-{(2R)-2-[(1E,3R)-4,4-difluoro-3-hydroxy-4-phenylbut-1-enyl]-5-oxopyrrolidin-1-yl}heptanoic acid (9a)

A mixture of compound **8a** (94 mg) and LiOH (0.25 mL, 1M) in MeOH (1 mL) was stirred at room temperature overight and concentrated in vacuo. The residue was co-evaporated with MeOH three times and the resudue was washed with diethyl ether three times (to remove trace of unhygolysed ester). After neutralizing with 1N HCl, the mixture was extracted with ethyl acetate (3x). The organic layer was washed with water and brine, dried over Na₂SO₄ and filtered. The filtrate was concentrated to give the title compound **9a**. MS (-ESI): m/z) 394.3 (M-1)⁻.

### EXAMPLE 10

### Isopropyl 7-{(2S)-2-[(3R)-4,4-difluoro-3-hydroxy-4-phenylbutyl]-5-oxopyrrolidin-1-yl}heptanoate (10a)

To a solution of **8a** (77 mg) in degassed MeOH was added Pd/C (12 mg, 10%) and the mixture was purged with hydrogen three times and then stirred under H₂ for 4.5h. After purging with nitrogen, the mixture was filtered through a celite pad and the filtrate was concentrated to give the desired title compound **10a**. ¹H NMR (400 MHz, acetone-d₆) δ 7.57-7.47 (m, 5H), 4.95 (m, 1H), 4.73 (d, 1H, OH), 4.06 (m, 1H), 3.65 (m, 1H), 3.60 (m, 1H), 2.90 (m, 1H), 2.25 (t, 2H), 2.25-2.10 (m, 3H), 1.90 (m, 1H), 1.70-1.20 (m, 12H), 1.20 (d, 6H).

### EXAMPLE 11

### 7-{(2S)-2-[(3R)-4,4-difluoro-3-hydroxy-4-phenylbutyl]-5-oxopyrrolidin-1-yl} heptanoic acid (11 a)

A mixture of compound **10a** (30 mg) and LiOH (0.8 mL, 1M) in MeOH (2.5 mL) was stirred at room temperature for three days and neutralized with 1N HCl. The MeOH was removed in vacuo and the residue extracted with ethyl acetate (3x). The organic layer was washed with water and brine, dried over Na₂SO₄ and filtered. The filtrated was concentrated to give the title compound **11a.** ¹H NMR (400 MHz, acetone-d₆) δ 7.57-7.47 (m, 5H), 4.80 (br s, 1H, OH), 4.06 (m, 1H), 3.68 (m, 1H), 3.52 (m, 1H), 2.91 (m, 1H), 2.28 (t, 2H), 2.25-2.10 (m, 3H), 1.90 (m, 1H), 1.70-1.20 (m, 12H).
I. Effects of an EP4 Agonist on Intraocular Pressure (IOP) in Rabbits and Monkeys.

### Animals

Drug-naïve, male Dutch Belted rabbits and female cynomolgus monkeys are used in this study. Animal care and treatment in this investigation are in compliance with guidelines by the National Institute of Health (NIH) and the Association for Research in Vision and Ophthalmology (ARVO) resolution in the use of animals for research. All experimental procedures str approved by the Institutional Animal Care and Use Committee of Merck and Company.

### Drug Preparation and Administration

Drug concentrations are expressed in terms of the active ingredient (base). The compounds of this invention are dissolved in physiological saline at 0.01, 0.001, 0.0001 % for rabbit study and 0.05, 0.005% for monkey studies. Drug or vehicle aliquots (25 ul) are administered topically unilaterally or bilaterally. In unilateral applications, the contralateral eyes receive an equal volume of saline. Proparacaine (0.5%) is applied to the cornea prior to tonometry to minimize discomfort. Intraocular pressure (IOP) is recorded using a pneumatic tonometer (Alcon Applanation Pneumatonograph) or equivalent.

### Statistical Analysis

The results are expressed as the changes in IOP from the basal level measured just prior to administration of drug or vehicle and represent the mean, plus or minus standard deviation. Statistical comparisons are made using the Student's t-test for non-paired data between responses of drug-treated and vehicle-treated animals and for paired data between ipsilateral and contralateral eyes at comparable time intervals. The significance of the date is also determined as the difference from the "t-0" value using Dunnett's "t" test. Asterisks represent a significance level of p<0.05.

### A. Intraocular Pressure Measurement in Rabbits

Male Dutch Belted rabbits weighing 2.5-4.0 kg are maintained on a 12-hour light/dark cycle and rabbit chow. All experiments are performed at the same time of day to minimize variability related to diurnal rhythm. IOP is measured before treatment then the compounds of this invention or vehicle are instilled (one drop of 25 ul) into one or both eyes and IOP is measured at 30, 60, 120, 180, 240, 300, and 360 minutes after instillation. In some cases, equal number of animals treated bilaterally with vehicle only are evaluated and compared to drug treated animals as parallel controls.

### B. Intraocular Pressure Measurements in Monkeys.

Unilateral ocular hypertension of the right eye is induced in female cynomolgus monkeys weighing between 2 and 3 kg by photocoagulation of the trabecular meshwork with an argon laser system (Coherent NOVUS 2000, Palo Alto, USA) using the method of Lee at al. (1985). The prolonged increase in intraocular pressure (IOP) results in changes to the optic nerve head that are similar to those found in glaucoma patients.

For IOP measurements, the monkeys are kept in a sitting position in restraint chairs for the duration of the experiment. Animals are lightly anesthetized by the intramuscular injection of ketamine hydrochloride (3-5 mg/kg) approximately five minutes before each IOP measurement and one drop of 0.5% proparacaine was instilled prior to recording IOP. IOP is measured using a pneumatic tonometer (Alcon Applanation Tonometer) or a Digilab pneumatonometer (Bio-Rad Ophthalmic Division, Cambridge, MA, USA).

IOP is measured before treatment and generally at 30, 60, 124, 180, 300, and 360 minutes after treatment. Baseline values are also obtained at these time points generally two or three days prior to treatment. Treatment consists of instilling one drop of 25 ul of the compounds of this invention (0.05 and 0.005 %) or vehicle (saline). At least one-week washout period is employed before testing on the same animal. The normotensive (contralateral to the hypertensive) eye is treated in an exactly similar manner to the hypertensive eye. IOP measurements for both eyes are compared to the corresponding baseline values at the same time point. Results are expressed as mean plus-or-minus standard deviation in mm Hg. The activity range of the compounds of this invention for ocular use is between 0.01 and 100,000 nM

By way of example, Compound 7 was a high affinity ligand at the EP4 receptor with a binding affinity in the range of 0.2-2 nM. It was also highly selective against other prostanoid receptors with binding affinities greater than 2 µM. In a PanLab screening against more than 80 receptors and enzymes, this compound displayed no significant activities at concentrations greater than 10 µM. The compound was a full agonist at the EP4 receptor with an EC₅₀ of 0.2 -10 nM in a number of cell based functional assays using standard methods for determining EP4 functional agonism. Compound 7 also had good oral bioavailability and terminal elimination half life in rats which are not known for prostaglandin analogs. This unique property allows for convenient oral dosing regimes for study EP4 agonism in in vivo models.

## Claims

1. Use, for the manufacture of a medicament for treating ocular hypertension or glaucoma, of a compound of formula I: 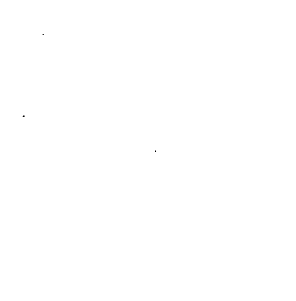 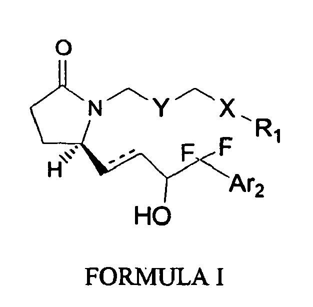 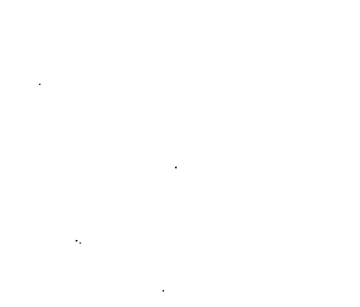 or a pharmaceutically acceptable salt, enantiomer, diastereomer, or mixture thereof, wherein,
X is (CH₂)ₙ, O or S;
Y represents (C(R^{b})₂)ₙ, triple bond, 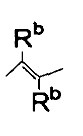 or 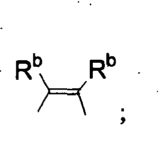 R₁ represents tetrazole which is unsubstituted or substituted with an Ra group, or (CH₂)ₚCO₂R₆, with the proviso that when X is a bond R₁ is not (CH₂)ₚCO₂R₆.
R² and R³ independently represent hydrogen, or C₁₋₄ alkyl;
R₆ represents hydrogen, or C₁₋₆ alkyl;
Ar₂ independently represents (CH₂)ₘC₆₋₁₀aryl, (CH₂)ₘC₅₋₁₀heteroaryl, (CH₂)ₘC₃₋₁₀ heterocycloalkyl, (CH₂)ₘC₃₋₈ cycloalkyl said cycloalkyl, heterocycloalkyl, aryl or heteroaryl unsubstituted or substituted with 1-3 groups of Rₐ;
Rₐ represents C₁₋₆ alkoxy, C₁₋₆ alkyl, CF₃, nitro, amino, cyano, C₁₋₆ alkylamino, or halogen;
R^{b} independently represents H, halogen, C₁₋₆ alkyl, or C₃₋₆ cylcoalkyl;
--- represents a double or single bond;
p represents 1-3;
n represents 0-4; and
m represents 0-8.

2. Use according to claim 1 wherein X and Y are (CH₂)ₙ,

3. Use according to claim 1 wherein Y is a double bond as
described by 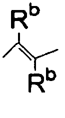 or 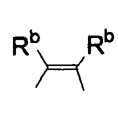 and all other variables are as originally described.

4. Use according to claim 1 wherein R₁ is (CH₂)ₚCO₂R₆, X is (CH₂)ₙ, and Y is (CH₂)ₙ.

5. Use according to claim 1 wherein Ar₂ is (CH₂)ₘC₆₋₁₀aryl, said aryl unsubstituted or substituted with 1 to 3 groups of R^{a} and all other variables are as originally described.

6. Use according to claim 1 wherein R₁ is a tetrazole unsubstituted or substituted with an Rₐ group X is (CH₂)ₙ, and Y is (CH₂)ₙ, C(halo)₂
or a double bond as described by 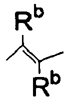 or

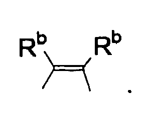

7. Use according to claim 1 wherein Ar₂ is a phenyl unsubstituted or substituted with 1 to 3 groups of Rₐ, R₁ is tetrazolyl, said tetrazolyl unsubstituted or substituted with a Rₐ group and all other variables are as originally described.

8. Use according to claim 1 wherein the compound is:
(5*R*)-5-[(1*E*)-3-hydroxy-4,4-difluoro-4-phenylbut-1-enyl]-1-{4-[(1-methyl-1*H-*tetrazol-5-yl)]butyl}pyrrolidin-2-one,
3-[4-{(2*R*)-2-[(1*E*)-3-hydroxy-4,4-difluoro-4-phenylbut-1-enyl]-5-oxopyrrolidin-1-yl}butyl)]propanoic acid,
(5*R*)-5-[(1*E*)-3-hydroxy-4,4-difluoro-4-phenylbut-1-enyl]-1-{4-[1*H*-tetrazol-5-ylmethyl)butyl}pyrrolidin-2-one,
[4-{(2*R*)-2-[(1*E*)-3-hydroxy-4,4-difluoro-4-phenylbut-1-enyl]-5-oxopyrrolidin-1-yl}butyl)]acetic acid,
(5*R*)-5-[(1*E*)-hydroxy-difluoro-4-phenylbut-1-enyl]-1-{4-[(1-methyl-1*H-*tetrazol-5-yl)thio]butyl}pyrrolidin-2-one,
(5*R*)-5-[(1*E*)-3-hydroxy-4,4-difluoro-4-phenylbut-1-enyl]-1-[4-(1*H*-tetrazol-5-ylthio)butyl]pyrrolidin-2-one,
3-[4-{(2*R*)-2-[(1*E*)-3-hydroxy-4,4-difluoro-4-phenylbut-1-enyl]-5-oxopyrrolidin-1-yl}butyl)thio]propanoic acid,
[4-{(2*R*)-2-[(1*E*)-3-hydroy-4,4-difluoro-4-phenylbut-1-enyl]-5-oxopyrrolidin-1-yl}butyl)thio]acetic acid,
(5*R*)-5[(1*E*)-4,4-difluoro-3-hydroxy-4-phenylbut-1-enyl]-1-[6-(1H-tetrazol-5-yl)hexyl]pyrrolidin-2-one ,
7-{(2*R*)-2-[(1*E*,3*R*)-4,4-difluoro-3-hydroxy-4-phenylbut-1-enyl]-5-oxopyrrolidin-1-yl}heptanoic acid,
isopropyl 7-{(2*R*)-2-[(1*E*,3*R*)-4,4-difluoro-3-hydroxy-4-phenylbut-1-enyl]-5-oxopyrrolidin-1-yl}heptanoate,
7-{(2*S*)-2-[(3*R*)-4,4-difluoro-3-hydroxy-4-phenylbutyl]-5-oxopyrrolidin-1-yl}heptanoic acid,
(5*Z*)-7-{(2*R*)-2-[(1*E*,3*R*)-4,4-difluoro-3-hydroxy-4-phenylbut-1-enyl]-5-oxopyrrolidin-1-yl}hept-5-enoic acid,
isopropyl (5*Z*)-7-{(2*R*)-2-[(1*E*,3*R*)-4,4-difluoro-3-hydroxy-4-phenylbut-1-enyl]-5-oxopyrrolidin-1-yl}hept-5-enoate,
7-{(2*R*)-2-[(1*E*,3*R*)-4-(3-chlorophenyl)-4,4-difluoro-3-hydroxybut-1-enyl]-5-oxopyrrolidin-1-yl}heptanoic acid,
isopropyl 7-{(2*R*)-2-[(1*E*,3*R*)-4-(3-chlorophenyl)-4,4-difluoro-3-hydroxybut-1-enyl]-5-oxopyrrolidin-1-yl}heptanoate,
7-((2*R*)-2-{(1*E*,3*R*)-4,4-difluoro-3-hydroxy-4-[3-(trifluoromethyl)phenyl]but-1-enyl}-5-oxopyrrolidin-1-yl)heptanoic acid,
isopropyl 7-((2*R*)-2-{(1*E*,3*R*)-4,4-difluoro-3-hydroxy-4-[3-(trifluoromethyl)phenyl]but-1-enyl}-5-oxopyrrolidin-1-yl)heptanoate,
cyclopentyl 7-{(2*R*)-2-[(1*E*,3*R*)-4,4-difluoro-3-hydroxy-4-phenylbut-1-enyl]-5-oxopyrrolidin-1-yl}heptanoate,
7-{(2*R*)-2-[(1*E*,3*R*)-4,4-difluoro-3-hydroxy-3-methyl-4-phenylbut-1-enyl]-5-oxopyrrolidin-1-yl}heptanoic acid,
isopropyl 7-{(2*R*)-2-[(1*E*,3*R*)-4,4-difluoro-3-hydroxy-3-methyl-4-phenylbut-1-enyl]-5-oxopyrrolidin-1-yl}heptanoate,
isobutyl 7-{(2*R*)-2-[(1*E*,3*R*)-4,4-difluoro-3-hydroxy-4-phenylbut-1-enyl]-5-oxopyrrolidin-1-yl}heptanoate,
cyclohexyl 7-{(2*R*)-2-[(1*E*,3*R*)-4,4-difluoro-3-hydroxy-4-phenylbut-1-enyl]-5-oxopyrrolidin-1-yl}heptanoate,
(5*R*)-5-[(1*E*)-(3R)-hydroxy-4,4-difluoro-4-phenylbut-1-enyl]-1-{4-[(1-methyl-1*H-*tetrazol-5-yl)]butyl} pyrrolidin-2-one,
3-[4-{(2*R*)-2-[(1*E*)-(3*R*)-hydroxy-4,4-difluoro-4-phenylbut-1-enyl]-5-oxopyrrolidin-1-yl}butyl)]propanoic acid,
(5*R*)-5-[(1*E*)-(3R)-hydroxy-4,4-difluoro-4-phenylbut-1-enyl]-1-{4-[1*H*-tetrazol-5-ylmethyl)butyl}pyrrolidin-2-one,
[4-{(2*R*)-2-[(1*E*)-(3R)-hydroxy-4,4-difluoro-4-phenylbut-1-enyl]-5-oxopyrrolidin-1-yl}butyl)]acetic acid,
(5*R*)-5-[(1*E*)-(3R)-hydroxy-4,4-difluoro-4-phenylbut-1-enyl]-1-{4-[(1-methyl-1*H-*tetrazol-5-yl)thio]butyl}pyrrolidin-2-one,
(5*R*)-5-[(1*E*)-(3R)-hydroxy-4,4-difluoro-4-phenylbut-1-enyl]-1-[4-(1*H*-tetrazol-5-ylthio)butyl]pyrrolidin-2-one,
3-[4-{(2*R*)-2-[(1*E*)-(3R)-hydroxy-4,4-difluoro-4-phenylbut-1-enyl]-5-oxopyrrolidin-1-yl}butyl)thio]propanoic acid,
[4-{(2*R*)-2-[(1*E*)-(3R)-hydroxy-4,4-difluoro-4-phenylbut-1-enyl]-5-oxopyrrolidin-1-yl}butyl)thio]acetic acid, or
(5R)-6[(1E)-4,4-difluoro-(3R)-hydroxy-4-phenylbut-1-enyl]-1-[6-(1H-tetrazol-5-yl)hexyl]pyrrolidin-2-one, a pharmaceutically acceptable salt, enantiomer, diastereomer, or mixture thereof.

9. Use, for the manufacture of a medicament for treating macular edema, macular degeneration, treating dry eye, increasing retinal and optic nerve head blood velocity or increasing retinal and optic nerve oxygen tension of a compound of formula I as recited in any of claims 1-8.

10. Use according to claim 1 or claim 9 wherein the medicament is a topical formulation as a solution or suspension.

11. Use according to claim 10 wherein a second active ingredient belonging to the group consisting of: β-adrenergic blocking agent, parasympathomimetic agent, sympathomimetic agent, carbonic anhydrase inhibitor, Maxi-K channel blocker, and a prostaglandin, hypotensive lipid, neuroprotectant, and 5-HT2 receptor agonist is added to the topical formulation.

12. Use according to claim 11 wherein the β-adrenergic blocking agent is timolol, betaxolol, levobetaxolol, carteolol, or levobunolol; the parasympathomimetic agent is pilocarpine; the sympathomimetic agent is epinephrine, brimonidine, iopidine, clonidine, or para-aminoclonidine, the carbonic anhydrase inhibitor is dorzolamide, acetazolamide, metazolamide or brinzolamide; the prostaglandin is latanoprost, travaprost, unoprostone, rescula, or S1033, the hypotensive lipid is lumigan, the neuroprotectant is eliprodil, R-eliprodil or memantine; and the 5-HT2 receptor agonist is 1-(2-aminopropyl)-3-methyl-1H-imdazol-6-ol fumarate or 2-(3-chloro-6-methoxy-indazol-1-yl)-1-methyl-ethylamine.

13. A compound of structural formula I: 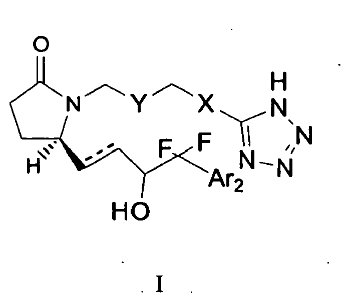 or a pharmaceutically acceptable salt, enantiomer, diastereomer, or mixture thereof, wherein
X is (CH₂)ₙ, O or S;
Y represents (C(R^{b})₂)ₙ, triple bond, 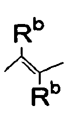 or 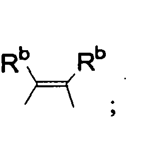 Ar₂ independently represent (CH₂)ₘC₆₋₁₀aryl, (CH₂)ₘC₅₋₁0heteroaryl, (CH₂)ₘC₃-₁₀ heterocycloalkyl, (CH₂)ₘC₃₋₈ cycloalkyl said cycloalkyl, heterocycloakyl, aryl or heteroaryl unsubstituted or substituted with 1-3 groups of Rₐ;
Rₐ represents C₁₋₆ alkoxy, C₁₋₆ alkyl, CF₃, nitro, amino, cyano, C₁₋₆ alkylamino, or halogen;
R^{b} independently represents H, halogen, C₁₋₆ alkyl, C₃₋₆ cylcoalkyl or
--- represents a double or single bond;
n represents 0-4; and
m represents 0-8.

14. The compound according to claim 13 wherein X and Y are (CH₂)ₙ, --- represents a double bond; and Ar₂ is phenyl.

15. The compound according to claim 14 wherein X is (CH₂)ₙ and n is 1 and Y is (CH₂)ₙ and n is 3.

16. A compound of claim 13, 14 or 15 for use in medicinal therapy.

## Patentansprüche

1. Verwendung einer Verbindung der Formel I: 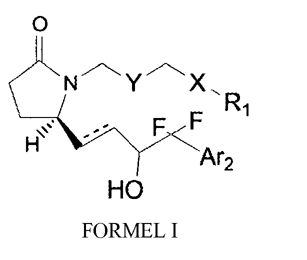 oder eines pharmazeutisch annehmbaren Salzes, Enantiomers, Diastereomers oder einer Mischung davon zur Herstellung eines Medikaments zur Behandlung von okulärer Hypertension oder Glaukom,
wobei
X (CH₂)ₙ, O oder S ist,
Y (C(R^{b})₂)ₙ, Dreifachbindung, 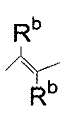 oder 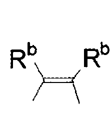 bedeutet,
R₁ Tetrazol, das unsubstituiert oder mit einer Ra-Gruppe substituiert ist, oder (CH₂)ₚCO₂R₆ bedeutet, mit der Maßgabe, dass, wenn X eine Bindung ist, R₁ nicht (CH₂)ₚCO₂R₆ ist,
R² und R³ unabhängig Wasserstoff oder C₁₋₄-Alkyl bedeuten,
R₆ Wasserstoff oder C₁₋₆-Alkyl bedeutet,
Ar₂ unabhängig (CH₂)ₘC₆₋₁₀-Aryl, (CH₂)ₘC₅₋₁₀-Heteroaryl, (CH₂)ₘC₃₋₁₀-Heterocycloalkyl, (CH₂)ₘC₃₋₈-Cycloalkyl bedeutet, wobei das Cycloalkyl, Heterocycloalkyl, Aryl oder Heteroaryl unsubstituiert oder mit 1-3 Rₐ-Gruppen substituiert ist,
Rₐ C₁₋₆-Alkoxy, C₁₋₆-Alkyl, CF₃, Nitro, Amino, Cyano, C₁₋₆-Alkylamino oder Halogen bedeutet,
R^{b} unabhängig H, Halogen, C₁₋₆-Alkyl oder C₃₋₆-Cycloalkyl bedeutet,
--- eine Doppel- oder Einfachbindung bedeutet,
p 1-3 bedeutet,
n 0-4 bedeutet und
m 0-8 bedeutet.

2. Verwendung gemäß Anspruch 1, wobei X und Y (CH₂)ₙ sind.

3. Verwendung gemäß Anspruch 1, wobei Y eine Doppelbindung ist, wie sie durch 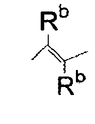 oder 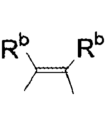 beschrieben ist, und alle anderen Variablen wie ursprünglich beschrieben sind.

4. Verwendung gemäß Anspruch 1, wobei R₁(CH₂)ₚCO₂R₆ ist, X (CH₂)ₙ ist und Y (CH₂)ₙ ist.

5. Verwendung gemäß Anspruch 1, wobei Ar₂(CH₂)ₘC₆₋₁₀-Aryl ist, das Aryl unsubstituiert oder mit 1 bis 3 R^{a}-Gruppen substituiert ist, und alle anderen Variablen wie ursprünglich beschrieben sind.

6. Verwendung gemäß Anspruch 1, wobei R₁ ein Tetrazol ist, unsubstituiert oder mit einer Rₐ-Gruppe substituiert, X (CH₂)ₙ ist und Y (CH₂)ₙ, C(Halogen)₂ oder eine Doppelbindung, wie sie durch 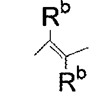 oder 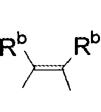 beschrieben ist, ist.

7. Verwendung gemäß Anspruch 1, wobei Ar₂ ein Phenyl ist, unsubstituiert oder mit 1 bis 3 Rₐ-Gruppen substituiert, R₁ Tetrazolyl ist, das Tetrazolyl unsubstituiert oder mit einer Rₐ-Gruppe substituiert ist, und alle anderen Variablen wie ursprünglich beschrieben sind.

8. Verwendung gemäß Anspruch 1, wobei die Verbindung ist:
(5*R*)-5-[(1*E*)-3-Hydroxy-4,4-difluor-4-phenylbut-1-enyl]-1-{4-[(1-methyl-1*H*-tetrazol-5-yl)]butyl}pyrrolidin-2-on,
3-[4-{(2*R*)-2-[(1*E*)-3-Hydroxy-4,4-difluor-4-phenylbut-1-enyl]-5-oxopyrrolidin-1-yl}butyl)]propansäure,
(5*R*)-5-[(1*E*)-3-Hydroxy-4,4-difluor-4-phenylbut-1-enyl]-1-{4-[1*H*-tetrazol-5-ylmethyl)butyl}pyrrolidin-2-on,
[4-{(2*R*)-2-[(1*E*-3-Hydroxy-4,4-difluor-4-phenylbut-2-enyl]-5-oxopyrrolidin-1-yl}butyl)]essigsäure,
(5*R*)-5-[(1*E*)-3-Hydroxy-4,4-difluor-4-phenylbut-1-enyl]-1-{4-[(1-methyl-1*H*-tetrazol-5-yl)thio]butyl}pyrrolidin-2-on,
(5*R*)-5-[(1*E*)-3-Hydroxy-4,4-difluor-4-phenylbut-1-enyl]-1-[4-(1*H*-tetrazol-5-ylthio)butyl]pyrrolidin-2-on, 3-[4-{(2*R*)-2-[(1*E*)-3-Hydroxy-4,4-difluor-4-phenylbut-1-enyl]-5-oxopyrrolidin-1-yl}butyl)thio]propansäure,
[4-{(2*R*)-2-[(1*E*)-3-Hydroxy-4,4-difluor-4-phenylbut-1-enyl]-5-oxopyrrolidin-1-yl}butyl)thio]essigsäure, (5*R*)-5[(1*E*)-4,4-Difluor-3-hydroxy-4-phenylbut-1-enyl]-1-[6-(1H-tetrazol-5-yl)hexyl]pyrrolidin-2-on, 7-{(2*R*)-2-[(1*E*,3*R*)-4,4-Difluor-3-hydroxy-4-phenylbut-1-enyl]-5-oxopyrrolidin-1-yl}heptansäure, Isopropyl-7-{(2*R*)-2-[(1*E*,3*R*)-4,4-difluor-3-hydroxy-4-phenylbut-1-enyl]-5-oxopyrrolidin-1-yl}heptanoat, 7-{(2)-2-[(3*R*)-4,4-Difluor-3-hydroxy-4-phenylbutyl]-5-oxopyrrolidin-1-yl}heptansäure, (5*Z*)-7-{(2*R*)-2-[(1*E*,3*R*)-4,4-Difluor-3-hydroxy-4-phenylbut-1-enyl]-5-oxopyrrolidin-1-yl}hept-5-ensäure, Isopropyl(5*Z*)-7-{(2*R*)-2-[(1*E*,3*R*)-4,4-difluor-3-hydroxy-4-phenylbut-1-enyl]-5-oxopyrrolidin-1-yl}hept-5-enoat,
7-{(2*R*)-2-[(1*E*,3*R*)-4-(3-Chlorphenyl)-4,4-difluor-3-hydroxybut-1-enyl]-5-oxopyrrolidin-1-yl}heptansäure, Isopropyl-7-{(2*R*)-2-[(1*E*,3*R*)-4-(3-chlorphenyl)-4,4-difluor-3-hydroxybut-1-enyl]-5-oxopyrrolidin-1-yl}heptanoat,
7-((2*R*)-2-{(1*E*,3*R*)-4,4-difluor-3-hydroxy-4-[3-(trifluormethyl)phenyl]but-1-enyl}-5-oxopyrrolidin-1-yl)heptansäure,
Isopropyl-7-((2*R*)-2-{(1*E*,3*R*)-4,4-difluor-3-hydroxy-4-[3-(trifluormethyl)phenyl]but-1-enyl}-5-oxopyrrolidin-1-yl)heptanoat,
Cyclopentyl-7-{(2*R*)-2-[(1*E*,3*R*)-4,4-difluor-3-hydroxy-4-phenylbut-1-enyl]-5-oxopyrrolidin-1-yl}heptanoat,
7-{(2*R*)-2-[(1*E*,3*R*)-4,4-Difluor-3-hydroxy-3-methyl-4-phenylbut-1-enyl]-5-oxopyrrolidin-1-yl}heptansäure,
Isopropyl-7-{(2*R*)-2-[(1*E*,3*R*)-4,4-difluor-3-hydroxy-3-methyl-4-phenylbut-1-enyl]-5-oxopyrrolidin-1-yl}heptanoat,
Isobutyl-7-{(2*R*)-2-[(1*E*,3*R*)-4,4-difluor-3-hydroxy-4-phenylbut-1-enyl]-5-oxopyrrolidin-1-yl}heptanoat, Cyclohexyl-7-{(2*R*)-2-[(1*E*,3*R*)-4,4-difluor-3-hydroxy-4-phenylbut-1-enyl]-5-oxopyrrolidin-1-yl}heptanoat,
(5*R*)-5-[(1*E*)-(3*R*)-Hydroxy-4,4-difluor-4-phenylbut-1-enyl]-1-{4-[(1-methyl-1*H*-tetrazol-5-yl)]butyl}pyrrolidin-2-on,
3-[4-{(2*R*)-2-[(1*E*)-(3*R*)-Hydroxy-4,4-difluor-4-phenylbut-1-enyl]-5-oxopyrrolidin-1-yl}butyl)]propansäure,
(5*R*)-5-[(1*E*)-(3*R*)-Hydroxy-4,4-difluor-4-phenylbut-1-enyl]-1-{4-[1*H*-tetrazol-5-ylmethyl)butyl}pyrrolidin-2-on,
[4-{(2*R*)-2-[(1*E*)-(3*R*)-Hydroxy-4,4-difluor-4-phenylbut-1-enyl]-5-oxopyrrolidin-1-yl}butyl)]essigsäure, (5*R*)-5-[(1*E*)-(3*R*)-Hydroxy-4,4-difluor-4-phenylbut-1-enyl]-1-{4-[(1-methyl-1*H*-tetrazol-5-yl)thio]butyl}pyrrolidin-2-on,
(5*R*)-5-[(1*E*)-(3*R*)-Hydroxy-4,4-difluor-4-phenylbut-1-enyl]-1-[4-(1*H*-tetrazol-5-ylthio)butyl]pyrrolidin-2-on,
3-[4-{(2*R*)-2-[(1*E*)-(3*R*)-Hydroxy-4,4-difluor-4-phenylbut-1-enyl]-5-oxopyrrolidin-1-yl}butyl)thio]propansäure,
[4-{(2*R*)-2-[(1*E*)-(3R)-Hydroxy-4,4-difluor-4-phenylbut-1-enyl]-5-oxopyrrolidin-1-yl}butyl)thio]essigsäure oder
(5R)-5-[(1E)-4,4-Difluor-(3R)-hydroxy-4-phenylbut-1-enyl]-1-[6-(1H-tetrazol-5-yl)hexyl]pyrrolidin-2-on,
ein pharmazeutisch annehmbares Salz, Enantiomer, Diastereomer oder eine Mischung davon.

9. Verwendung einer wie in einem der Ansprüche 1-8 genannten Verbindung der Formel I zur Herstellung eines Medikaments zur Behandlung von Makulaödem, Makuladegeneration, Behandlung von trockenem Auge, Erhöhung der Blutflussgeschwindigkeit in der Netzhaut und dem Sehnervenkopf und des Sehnervenkopf-Sauerstoffpartialdrucks.

10. Verwendung gemäß Anspruch 1 oder Anspruch 9, wobei das Medikament eine topische Formulierung als eine Lösung oder Suspension ist.

11. Verwendung gemäß Anspruch 10, wobei ein zweiter Wirkstoff zu der topischen Formulierung zugegeben wird, der zur Gruppe gehört, bestehend aus: β-adrenergem Blocker, Parasympathomimetikum, Sympathomimetikum, Carboanhydraseinhibitor, Maxi-K-Kanal-Blocker und einem Prostaglandin, hypotonischen Lipid, Neuroprotektor und 5-HT2-Rezeptor-Agonist.

12. Verwendung gemäß Anspruch 11, wobei der β-adrenerge Blocker Timolol, Betaxolol, Levobetaxolol, Carteolol oder Levobunolol ist, das Parasympathomimetikum Pilocarpin ist, das Sympathomimetikum Epinephrin, Brimonidin, Iopidin, Clonidin oder para-Aminoclonidin ist, der Carboanhydraseinhibitor Dorzolamid, Acetazolamid, Metazolamid oder Brinzolamid ist, das Prostaglandin Latanoprost, Travaprost, Unoproston, Rescula oder S1033 ist, das hypotonische Lipid Lumigan ist, der Neuroprotektor Eliprodil, R-Eliprodil oder Memantin ist und der 5-HT2-Rezeptor-Agonist 1-(2-Aminopropyl)-3-methyl-1H-imidazol-6-ol-fumarat oder 2-(3-Chlor-6-methoxyindazol-1-yl)-1-methylethylamin ist.

13. Eine Verbindung der Strukturformel I: 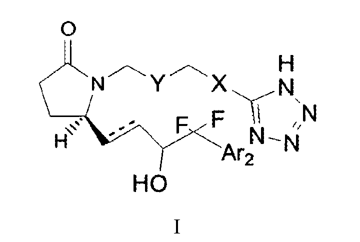 oder ein pharmazeutisch annehmbares Salz, Enantiomer, Diastereomer oder eine Mischung davon,
wobei
X (CH₂)ₙ, O oder S ist,
Y (C(R^{b})₂)ₙ, Dreifachbindung, 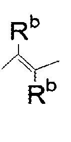 oder 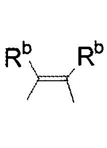 bedeutet,
Ar₂ unabhängig (CH₂)ₘC₆₋₁₀-Aryl, (CH₂)ₘC₅₋₁₀-Heteroaryl, (CH₂)ₘC₃₋₁₀-Heterocycloalkyl, (CH₂)ₘC₃₋₈-Cycloalkyl bedeutet, wobei das Cycloalkyl, Heterocycloalkyl, Aryl oder Heteroaryl unsubstituiert oder mit 1-3 Rₐ-Gruppen substituiert ist,
Rₐ C₁₋₆-Alkoxy, C₁₋₆-Alkyl, CF₃, Nitro, Amino, Cyano, C₁₋₆-Alkylamino oder Halogen bedeutet,
R^{b} unabhängig H, Halogen, C₁₋₆-Alkyl oder C₃₋₆-Cycloalkyl bedeutet oder
--- eine Doppel- oder Einfachbindung bedeutet,
n 0-4 bedeutet und
m 0-8 bedeutet.

14. Die Verbindung gemäß Anspruch 13, wobei X und Y (CH₂)ₙ sind, ---- eine Doppelbindung bedeutet und Ar₂ Phenyl ist.

15. Die Verbindung gemäß Anspruch 14, wobei X (CH₂)ₙ ist und n 1 ist und Y (CH₂)ₙ ist und n 3 ist.

16. Eine Verbindung gemäß Anspruch 13, 14 oder 15 zur Verwendung in der medizinischen Therapie.

## Revendications

1. Utilisation, pour la fabrication d'un médicament pour le traitement d'une hypertension oculaire ou d'un glaucome, d'un composé de la formule I: 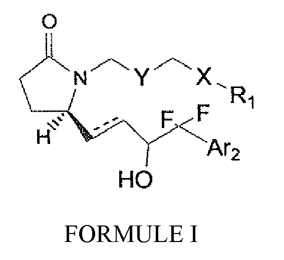 ou d'un sel pharmaceutiquement acceptable, d'un énantiomère, d'un diastéréoisomère ou d'un mélange de ceux-ci, dans laquelle:
X est (CH₂)ₙ, O ou S;
Y représente (C(R^{b})₂)ₙ, une liaison triple, 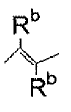 ou 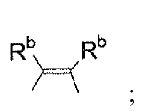
R₁ représente un tétrazole qui est non substitué ou substitué avec un groupe Rₐ, ou (CH₂)ₚCO₂R₆, sous réserve que lorsque X est une liaison, R₁ ne soit pas (CH₂)ₚCO₂R₆, R² et R³ représentent indépendamment un hydrogène ou un alkyle C₁₋₄;
R₆ représente un hydrogène ou un alkyle C₁₋₆;
Ar₂ représente indépendamment un (CH₂)ₘaryle C₆₋₁₀, un (CH₂)ₘhétéroaryle C₅₋₁₀, un (CH₂)ₘhétérocycloalkyle C₃₋₁₀, un (CH₂)ₘcycloalkyle C₃₋₈, ledit cycloalkyle, hétérocycloalkyle, aryle ou hétéroaryle étant non substitué ou substitué avec 1-3 groupes de Rₐ;
Rₐ représente un alcoxy C₁₋₆, un alkyle C₁₋₆, CF₃, un nitro, un amino, un cyano, un alkylamino C₁₋₆ ou un halogène;
R^{b} représente indépendamment H, un halogène, un alkyle C₁₋₆ ou un cycloalkyle C₃₋₆;
--- représente une liaison double ou simple;
p représente 1-3;
n représente 0-4; et
m représente 0-8.

2. Utilisation selon la revendication 1, dans laquelle X et Y sont (CH₂)ₙ.

3. Utilisation selon la revendication 1, dans laquelle Y est une liaison double comme il est décrit par: 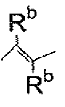 ou 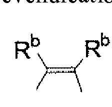 et toutes les autres variables sont comme il a été décrit à l'origine.

4. Utilisation selon la revendication 1, dans laquelle R₁ est (CH₂)ₚCO₂R₆, X est (CH₂)ₙ et Y est (CH₂)ₙ.

5. Utilisation selon la revendication 1, dans laquelle Ar₂ est un (CH₂)ₘaryle C₆₋₁₀, ledit aryle étant non substitué ou substitué avec 1-3 groupes de Rₐ et toutes les autres variables sont comme il a été décrit à l'origine.

6. Utilisation selon la revendication 1, dans laquelle R₁ est un tétrazole non substitué ou substitué avec un groupe Rₐ, X est (CH₂)ₙ et Y est (CH₂)ₙ, C(halo)₂ ou une liaison double comme il est décrit par:

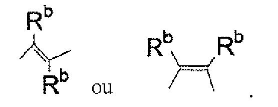

7. Utilisation selon la revendication 1, dans laquelle Ar₂ est un phényle non substitué ou substitué avec de 1 à 3 groupes de Rₐ, R₁ est un tétrazolyle, ledit tétrazolyle étant non substitué ou substitué avec un groupe Rₐ et toutes les autres variables sont comme il a été décrit à l'origine.

8. Utilisation selon la revendication 1, dans laquelle le composé est:
la (5R)-5-[(1E)-3-hydroxy-4,4-difluoro-4-phénylbut-1-ényl]-1-{4-[(1-méthyl-1H-tétrazol-5-yl)]butyl}pyrrolidin-2-one,
l'acide 3-[4-{(2R)-2-[(1E)-3-hydroxy-4,4-difluoro-4-phénylbut-1-ényl]-5-oxopyrrolidin-1-yl}butyl]propanoïque,
la (5R)-5-[(1E)-3-hydroxy-4,4-difluoro-4-phénylbut-1-ényl]-1-{4-[1H-tétrazol-5-ylméthyl]butyl}-pyrrolidin-2-one,
l'acide [4-{(2R)-2-[(1E)-3-hydroxy-4,4-difluoro-4-phénylbut-1-ényl]-5-oxopyrrolidin-1-yl}-butyl]acétique,
la (5R)-5-[(1E)-3-hydroxy-4,4-difluoro-4-phénylbut-1-ényl]-1-{4-[(1-méthyl-1H-tétrazol-5-yl)thio]butyl}pyrrolidin-2-one,
la (5R)-5-[(1E)-3-hydroxy-4,4-difluoro-4-phénylbut-1-ényl]-1-[4-(1H-tétrazol-5-ylthio)butyl]-pyrrolidin-2-one,
l'acide 3-[4-{(2R)-2-[(1E)-3-hydroxy-4,4-difluoro-4-phénylbut-1-ényl]-5-oxopyrrolidin-1-yl}butyl)thio]propanoïque,
l'acide [4-{(2R)-2-[(1E)-3-hydroxy-4,4-difluoro-4-phénylbut-1-ényl]-5-oxopyrrolidin-1-yl}-butyl)thio]acétique,
la (5R)-5-[(1E)-4,4-difluoro-3-hydroxy-4-phénylbut-1-ényl]-1-[6-(1H-tétrazol-5-yl)hexyl]-pyrrolidin-2-one,
l'acide 7-{(2R)-2-[(1E,3R)-4,4-difluoro-3-hydroxy-4-phénylbut-1-ényl]-5-oxopyrrolidin-1-yl}-heptanoïque,
le 7-{(2R)-2-[(1E,3R)-4,4-difluoro-3-hydroxy-4-phénylbut-1-ényl]-5-oxopyrrolidin-1-yl}-heptanoate d'isopropyle,
l'acide 7-{(2S)-2-[(3R)-4,4-difluoro-3-hydroxy-4-phénylbutyl]-5-oxopyrrolidin-1-yl}-heptanoïque,
l'acide (5Z)-7-{(2R)-2-[(1E,3R)-4,4-difluoro-3-hydroxy-4-phénylbut-1-ényl]-5-oxopyrrolidin-1-yl}hept-5-énoïque,
le (5Z)-7-{(2R)-2-[(1E,3R)-4,4-difluoro-3-hydroxy-4-phénylbut-1-ényl]-5-oxopyrrolidin-1-yl}-hept-5-énoate d'isopropyle,
l'acide 7-{(2R)-2-[(1E,3R)-4-(3-chlorophényl)-4,4-difluoro-3-hydroxybut-1-ényl]-5-oxo-pyrrolidin-1-yl}heptanoïque,
le 7-{(2R)-2-[(1E,3R)-4-(3-chlorophényl)-4,4-difluoro-3-hydroxybut-1-ényl]-5-oxopyrrolidin-1-yl}heptanoate d'isopropyle,
l'acide 7-((2R)-2-{(1E,3R)-4,4-difluoro-3-hydroxy-4-[3-(trifluorométhyl)phényl]but-1-ényl}-5-oxopyrrolidin-1-yl)heptanoïque,
le 7-((2R)-2-{(1E,3R)-4,4-difluoro-3-hydroxy-4-[3-(trifluorométhyl)phényl]but-1-ényl}-5-oxopyrrolidin-1-yl)heptanoate d'isopropyle,
le 7-{(2R)-2-[(1E,3R)-4,4-difluoro-3-hydroxy-4-phénylbut-1-ényl]-5-oxopyrrolidin-1-yl}-heptanoate de cyclopentyle,
l'acide 7-{(2R)-2-[(1E,3R)-4,4-difluoro-3-hydroxy-3-méthyl-4-phénylbut-1-ényl]-5-oxo-pyrrolidin-1-yl}-heptanoïque,
le 7-{(2R)-2-[(1E,3R)-4,4-difluoro-3-hydroxy-3-méthyl-4-phénylbut-1-ényl]-5-oxopyrrolidin-1-yl}heptanoate d'isopropyle,
le 7-{(2R)-2-[(1E,3R)-4,4-difluoro-3-hydroxy-4-phénylbut-1-ényl]-5-oxopyrrolidin-1-yl}heptanoate d'isobutyle,
le 7-{(2R)-2-[(1E,3R)-4,4-difluoro-3-hydroxy-4-phénylbut-1-ényl]-5-oxopyrrolidin-1-yl}heptanoate de cyclohexyle,
la (5R)-5-[(1E)-(3R)-hydroxy-4,4-difluoro-4-phénylbut-1-ényl]-1-{4-[(1-méthyl-1H-tétrazol-5-yl)]butyl}pyrrolidin-2-one,
l'acide 3-[4-{(2R)-2-[(1E)-(3R)-hydroxy-4,4-difluoro-4-phénylbut-1-ényl]-5-oxopyrrolidin-1-yl}butyl]propanoïque,
la (5R)-5-[(1E)-(3R)-hydroxy-4,4-difluoro-4-phénylbut-1-ényl]-1-{4-[1H-tétrazol-5-ylméthyl]-butyl}pyrrolidin-2-one,
l'acide [4-{(2R)-2-[(1E)-(3R)-hydroxy-4,4-difluoro-4-phénylbut-1-ényl]-5-oxopyrrolidin-1-yl}butyl]acétique,
la (5R)-5-[(1E)-(3R)-hydroxy-4,4-difluoro-4-phénylbut-1-ényl]-1-{4-[(1-méthyl-1H-tétrazol-5-yl)thio]butyl}pyrrolidin-2-one,
la (5R)-5-[(1E)-(3R)-hydroxy-4,4-difluoro-4-phénylbut-1-ényl]-1-[4-(1H-tétrazol-5-ylthio)-butyl]pyrrolidin-2-one,
l'acide 3-[4-{(2R)-2-[(1E)-(3R)-hydroxy-4,4-difluoro-4-phénylbut-1-ényl]-5-oxopyrrolidin-1-yl}butyl)thio]propanoïque,
l'acide [4-{(2R)-2-[(1E)-(3R)-hydroxy-4,4-difluoro-4-phénylbut-1-ényl]-5-oxopyrrolidin-1-yl}butyl)thio]acétique, ou
la (5R)-5-[(1E)-4,4-difluoro-(3R)-hydroxy-4-phénylbut-1-ényl]-1-[6-(1H-tétrazol-5-yl)hexyl]-pyrrolidin-2-one, un sel phamaceutiquement acceptable, un énantiomère, un diastéréoisomère ou un mélange de ceux-ci.

9. Utilisation, pour la fabrication d'un médicament pour le traitement d'un oedème maculaire, d'une dégénérescence maculaire, le traitement de l'oeil sec, l'augmentation de la vitesse sanguine de la rétine et de la tête du nerf optique ou l'augmentation de la tension d'oxygène de la rétine et du nerf optique, d'un composé de la formule I telle qu'énoncée dans l'une quelconque des revendications 1-8.

10. Utilisation selon la revendication 1 ou la revendication 9, dans laquelle le médicament est une formulation topique sous forme d'une solution ou d'une suspension.

11. Utilisation selon la revendication 10, dans laquelle un deuxième ingrédient actif appartenant au groupe constitué de: un agent bloquant β-adrénergique, un agent parasympathomimétique, un agent sympathomimétique, un inhibiteur d'anhydrase carbonique, un agent bloquant les canaux maxi-K et une prostaglandine, un lipide hypotenseur, un neuroprotecteur et un agoniste du récepteur 5-HT2 est ajouté à la formulation topique.

12. Utilisation selon la revendication 11, dans laquelle l'agent bloquant β-adrénergique est le timolol, le bétaxolol, le lévobétaxolol, le cartéolol ou le lévobunolol; l'agent parasympathomimétique est la pilocarpine; l'agent sympathomimétique est l'épinéphrine, la brimonidine, l'iopidine, la clonidine ou la para-aminoclonidine; l'inhibiteur d'anhydrase carbonique est le dorzolamide, l'acétazolamide, le métazolamide ou le brinzolamide; la prostaglandine est le latanoprost, le travaprost, l'unoprostone, le rescula ou S1033, le lipide hypotenseur est le lumigan; le neuroprotecteur est l'éliprodil, le R-éliprodil ou la mémantine; et l'agoniste du récepteur 5-HT2 est le fumarate de 1-(2-aminopropyl)-3-méthyl-1H-indazol-6-ol ou la 2-(3-chloro-6-méthoxy-indazol-1-yl)-1-méthyl-éthylamine.

13. Composé de la formule structurale I: 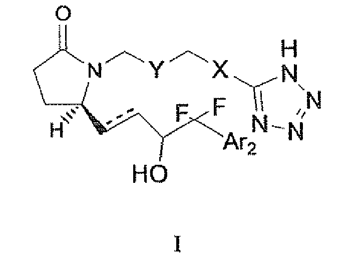 ou un sel pharmaceutiquement acceptable, un énantiomère, un diastéréoisomère ou un mélange de ceux-ci, dans lequel:
X est (CH₂)ₙ, O ou S;
Y représente (C(R^{b})₂)ₙ, une liaison triple, 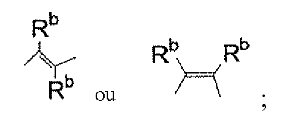
Ar₂ représente indépendamment un (CH₂)ₘaryle C₆₋₁₀, un (CH₂)ₘhétéroaryle C₅₋₁₀, un (CH₂)ₘhétérocycloalkyle C₃₋₁₀, un (CH₂)ₘcycloalkyle C₃₋₈, ledit cycloalkyle, hétérocycloalkyle, aryle ou hétéroaryle étant non substitué ou substitué avec 1-3 groupes de Rₐ;
Rₐ représente un alcoxy C₁₋₆, un alkyle C₁₋₆, CF₃, un nitro, un amino, un cyano, un alkylamino C₁₋₆ ou un halogène;
R^{b} représente indépendamment H, un halogène, un alkyle C₁₋₆ ou un cycloalkyle C₃-₆;
--- représente une liaison double ou simple;
n représente 0-4; et
m représente 0-8.

14. Composé selon la revendication 13, dans lequel X et Y sont (CH₂)ₙ; --- représente une liaison double; et Ar₂ est un phényle.

15. Composé selon la revendication 14, dans lequel X est (CH₂)ₙ et n est 1 et Y est (CH₂)ₙ et n est 3.

16. Composé selon la revendication 13, 14 ou 15 pour une utilisation dans une thérapie médicale.
